(19) **Europäisches Patentamt European Patent Office Office européen des brevets**

(11) **EP 1 778 194 B1**

(12) **EUROPEAN PATENT SPECIFICATION**

(45) Date of publication and mention of the grant of the patent:
**08.04.2015 Bulletin 2015/15**

(21) Application number: **05773151.5**

(22) Date of filing: **27.07.2005**

(51) Int Cl.:
***A61K 9/16*** (2006.01)

(86) International application number:
**PCT/GB2005/002930**

(87) International publication number:
**WO 2006/010921 (02.02.2006 Gazette 2006/05)**

(54) **PROCESS FOR PREPARING MICROCRYSTALS**

VERFAHREN ZUR HERSTELLUNG VON MIKROKRISTALLEN

PROCEDE POUR PREPARER DES MICROCRISTAUX

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.07.2004 GB 0416694**

(43) Date of publication of application:
**02.05.2007 Bulletin 2007/18**

(73) Proprietor: **University of Strathclyde Glasgow G1 1YQ, Scotland (GB)**

(72) Inventors:
• **MOORE, Barry, Douglas Crosshill Glasgow G42 8DT (GB)**
• **VOS, Jan Glasgow G5 9JG (GB)**

(74) Representative: **Edwards, Fiona Anne HGF Limited Delta House 50 West Nile Street Glasgow G1 2NP (GB)**

(56) References cited:
**WO-A-03/002225        WO-A-2004/062560 US-A- 5 662 883**

**Description**

**Field of the Invention**

This invention relates if general to micron or sub-micron particles comprising one or more water-soluble crystals wherein the crystals have a surface coating comprising one or more bioactive molecules as well as efficient methods of forming such particles and rapid methods for screening preferred conditions to form such particles. The particles are suitable for pharmaceutical formulations.

**Background of the Invention**

WO 00/69887 is a previous application by the present inventors which relates to protein coated microcrystals (PCMCs). The coated crystals disclosed in WO 00/69887 are generally coprecipitated from an aqueous mixture containing a saturated solution of a coprecipitant and a biomclecule by addition to a water miscible solvent. However, there is no disclosure that it would be advantageous to use a less than saturated solution.

US-A-5,662,883 relates to batch, semi-continuous and continuous processes which are carried out in a basic environment and require neutralisation to produce dispersed product.

In WO 00/69887 production of PCMCs by addition of a saturated aqueous solution of coprecipitant and the bioactive molecule to excess solvent is described. The preferred method in WO 00/69887 for obtaining efficient admixing was to dropwise add the aqueous solution to an excess of organic miscible solvent with vigorous mixing. However, this batch type process suffers from a number of drawbacks:

a) The precipitation conditions are continuously varying because the water content of the solvent is increasing throughout. It has been found that different initial water content leads to different sizes and shapes of crystals and to variations in bioactivity;

b) The precipitation is carried out into a suspension that contains an increasing quantity of crystals already in suspension. This will enhance the likelihood of nascent crystals fusing onto already formed crystals;

c) If a large-scale batch is required it is difficult to obtain high efficiency agitation with stirred batch reactors without excessive shear forces. High efficiency agitation is generally required to produce smaller crystals and prevent 'cementing' of crystals into aggregates. However, high shear forces can initiate damage to the bioactive molecule such as protein denaturation or 'nicking' of nucleic acids. Alternative approaches to rapid mixing such as nebulising the aqueous inflow to provide very small droplets also have potential problems arising from shear forces and interfacial denaturation processes;

d) The bioactive molecule and the coprecipitant require to be prepared and stored as a mixture until added to solvent. This can cause problems if, for example, a biomolecule is unstable in the mixture or else it requires the presence of additives or stabilisers, for example, to prevent aggregation, precipitation or chemical modification. If these need to be present above a threshold concentration they may interfere with the coprecipitation process;

e) It is difficult to put in place an automated screening procedure for determining optimum conditions for carrying out the coprecipitation process such that bioactive coated microcrystals with the desired physical properties and optimal bioactivity are produced. This arises because once the coprecipitant and bioactive molecule are mixed together many of the parameters that effect the coprecipitation process take up a fixed value and cannot be varied relative to each other. For example the aqueous-solvent ratio, the concentration of coprecipitant used and the loading of bioactive molecule in the particle cannot be varied relative to each other without firstly preparing further aqueous mixtures. This is time-consuming, inefficient and may introduce errors. Further if the bioactive molecule is only available in low quantities and/or is expensive to produce then preparation of many different aqueous solutions may become impossible or else be uneconomic. Relevant physical properties that require to be screened include size, shape, crystallinity, Zeta potential, aerodynamic properties, solubility and flowability. Factors effecting bioactivity include yield and loading of the bioactive molecule onto the microcrystal, water content and changes to the bioactive molecule structure, composition and aggregation state. The large number of variable parameters mean there is a clear need for new efficient methods which allow for screening of the best conditions to produce particles which have the desired physical properties with optimal bioactivity. For example, this would allow pharmaceutical formulations to be optimised more rapidly.

Although continuous methods for making dry protein powders have been disclosed that use supercritical fluids, these methods do not provide protein coated microcrystals and suffer from the disadvantage that they require the use of specialised high pressure pumping systems. In addition, because supercritical carbon dioxide and water are only miscible over a narrow range, it is necessary to employ a third solvent and use sophisticated mixing devices that are able to take advantage of the low viscosity and high diffusivity of supercritical fluids. One further serious problem is that, in the presence of water, supercritical carbon dioxide becomes acidic and hence this method is not well suited for processing the many bioactive molecules that are sensitive to low pH.

It is clear therefore that there is a need to develop alternative continuous processes for preparing protein powders which: a) can be applied to a wider range of bioactive molecules; b) can be applied to conventional solvents; c) are easier and less expensive to operate; and d) can be used for production of protein coated microcrystals.

It is an object of at least one aspect of the present invention to obviate or mitigate at least one or more of the aforementioned disadvantages.

## Summary of the Invention

According to a first aspect of the present invention there is provided a continuous method of forming bioactive molecule coated microcrystals comprising the following steps:

(a) providing a first aqueous solution comprising coprecipitant molecules;
(b) providing a second aqueous solution comprising bioactive molecules;
(c) providing a third solution comprising water miscible solvent;
(d) mixing said first aqueous solution, said second aqueous solution and said third solution simultaneously; wherein a first pump continuously delivers the first aqueous solution comprising coprecipitant molecules, a second pump continuously delivers the second aqueous solution comprising bioactive molecules and a third pump continuously delivers the third solution comprising water miscible solvent, such that coprecipitation of the coprecipitant and the bioactive molecules is initiated leading to formation of said microcrystals; and
(e) collecting a suspension of microcrystals.

It has surprisingly been found that it is highly advantageous to provide separate solutions for the first aqueous solution comprising coprecipitant molecules, the second aqueous solution comprising bioactive molecules and the third solution comprising water miscible solvent. For example, providing the coprecipitant molecules and the bioactive molecules as two separate solutions advantageously enables these components to be stored and introduced into a coprecipitation process from solutions containing different additives and/or pH and for the solutions to be held at different temperatures and pressures.

By continuous process herein means a process in which the prepared solutions are constantly mixed. The solutions may be constantly mixed, for example, by pumping the separate solutions into a mixing device or mixing devices. The at least three separate solutions may be mixed together substantially at the same time within one device.

The combined mixture may be simultaneously pumped out of the mixing device(s) until sufficient quantities of microcrystals have been formed.

In scale-up processes for manufacturing formulations of microcrystals with a surface coating comprising one or more bioactive molecules, it may be desirable to maximise the yield of bioactive molecules bound to carrier crystals. This is because it may be very costly to manufacture and purify, for example, therapeutic proteins. It may therefore be desirable to minimise the amount of bioactive molecules lost during immobilisation onto carrier crystals. The loss may arise either from unbound bioactive molecules remaining in the miscible solvent in the form of sub-micron particles or else from loss of bioactivity of the bound bioactive molecules. The efficiency of the binding process may be tested by running the coprecipitation process for a fixed period, such that a known quantity of bioactive molecules has been introduced, then isolating the formed microcrystals from the precipitation solvent by filtration and measuring the percentage of introduced bioactive molecules that is bound in the formed microcrystals. Typically, the efficiency of binding of the bioactive molecules to the carrier crystals may be greater than about 20%, greater than about 50%, greater than about 80% and more preferably greater than about 90%.

It has surprisingly been found that the efficiency of binding of bioactive molecules such as proteins during coprecipitation may be particularly sensitive to the pH of the aqueous solutions and concentration of introduced salt or buffer. At low salt concentrations such as less than 0.1M or preferably less than 0.01M, the binding efficiency may generally be found to improve if the pH of the aqueous solution used in the coprecipitation is moved closer to the pI of the bioactive molecules such as protein. Without wishing to be bound by theory, it may be that bioactive molecules with a reduced overall charge may be more able to cluster into nanoparticles and these can also then pack better together to coat the surface of carrier crystals.

It has also been discovered that the pH may also effect the amount of bound bioactive molecules that may be successfully reconstituted, for example, with antibodies such as IgG. In such cases, it may also be desirable to optimise the coprecipitation pH.

Unfortunately, when the previously disclosed two-line continuous process described in WO 00/69887 is used to carry out the coprecipitation process, then changing the pH of the aqueous solution may present difficulties particularly if the coprecipitant to be used has a buffering capacity - for example an amino acid. In such cases, it may be necessary to introduce significant quantities of a pH controlling agent such as an acid, base or buffer to move the concentrated coprecipitant solution towards a pH close to the pI of the bioactive modules. The presence of these controlling agents at high concentrations may interfere with the precipitation process and lead to a reduced amount of carrier material precipitating. The presence of large amounts of the controlling agents in the final solid formulation may also often be undesirable. For example, on reconstitution solutions with undesirable tonicity, unfavourable osmotic or ionic strengths above acceptable regulatory limits may be obtained such that they are not suitable for parenteral administration. It is therefore clear that although changes in pH can improve the situation, the two-line process described in WO 00/69887 may not provide an adequate route to commercial requirements to prepare microcrystals with a surface coating comprising one or more bioactive molecules at high efficiency. This will be particularly so for bioactive molecules with a pI far from the pI of a chosen coprecipitant.

Surprisingly, it has now been found that difficulties relating to obtaining an efficient manufacturing process may be overcome if coprecipitant and bioactive molecule solutions of different pH may be introduced as separate streams into a continuous mixing device and rapidly combined with water miscible solvent. This process which may be called a three-line process may be operated with the aqueous coprecipitant and bioactive molecule solutions at different pH which may significantly improve the efficiency of bioactive molecule binding such as protein binding compared to use of a two-line process operated without buffering. The process may also be used to obtain higher levels of, for example, soluble antibodies without the need to buffer the coprecipitant solution. The mixing of the three-streams may be carried out substantially at the same time or else one or other of the aqueous streams may be combined first with the miscible solvent and then this mixture rapidly combined with the other aqueous stream. The improved binding observed is surprising because it would be expected that exchange of protons between the components in the two aqueous streams would be very rapid on mixing and any initial differences between the streams would therefore be quickly eliminated.

In the present invention, the first aqueous solution comprising coprecipitant molecules may be introduced into a mixing process at a higher rate than the second aqueous solution comprising bioactive molecules with the third solution comprising water miscible solvent introduced at an even higher rate. The relative rates of addition may be altered as required to provide for a specific final composition and water content within the formed suspension of microcrystals.

The pH of the first aqueous solution comprising coprecipitant molecules may be chosen to provide rapid precipitation of well formed coprecipitant microcrystals. In the case of zwitterionic compounds, the pH optimum may be commonly around the pH where the overall neutral molecule exists in solution. For amino-acids, the appropriate pH may be conveniently achieved by dissolution of solid crystals of the pure compound into water to make a concentrated solution at about the saturated concentration.

The second aqueous solution comprising bioactive molecules may be buffered to a pH that may be different from the first aqueous solution comprising coprecipitant molecules and closer to the pI of the bioactive molecule using, for example, physiologically acceptable buffers. The buffer concentration may be selected from any of less than about 0.1 M, less than about 0.05 M, and preferably less than about 0.02 M. The pH may preferably be chosen such that the bioactive molecule remains soluble in the aqueous solution and retain the bioactivity of the bioactive molecule over the holding period prior to precipitation. To maximise the efficiency of binding, the pH used for the second aqueous solution comprising bioactive molecules, may generally be within 4.5 pH units of its pI, preferably within 3 pH units of its pI and most preferably within 2 pH units of its pI. As a concentration of the bioactive molecule is increased, it may be necessary to move further from the pI to prevent precipitation or aggregation of the bioactive molecules. It will be clear to a person skilled in the art that the amount of buffer components required to keep, for example, a therapeutic protein solution at a particular pH will be much less than that required to buffer a larger volume of a concentrated coprecipitant solution. This will be particularly advantageous for ionisable coprecipitants such as, for example, an amino acid.

The present invention therefore provides a method of increasing the efficiency of the coprecipitation process, while minimising the proportion of buffer compounds introduced into the final formed microcrystals. Similarly, separating the bioactive molecule solutions form the coprecipitant solutions may also lower the amount of other additives such as salts, surfactants, stabilisers and anti-oxidants introduced into the microcrystals compared to that of a two-line process as described in WO 00/69887.

In certain applications such as for post-precipitation coating, mixed coprecipitant systems or cross-linking it may be necessary to prepare, pump and mix in further solutions via additional inlet ports or via other mixing device placed in series before or after the coprecipitation device. Similarly, for screening applications an additional aqueous make-up stream can advantageously be used to allow initial or final concentrations of the coprecipitant and bioactive molecule to be varied in a systematic way. It should also be understood that although the majority of the application refers to a three-stream mixing process, any number more than three is also meant to be within the scope of the present application. For example, a four-stream, five-stream, six-stream, seven-stream, eight-stream, nine-stream or ten-stream process may be used.

A preferred feature of the continuous process described is that the combined flow-rate through a mixing device is high so that the dwell time of the combined solutions in the mixing device is very small. It is also preferable to ensure substantially complete mixing occurs in each device. The dwell-time may be calculated as the volume of the mixing chamber divided by the total volume of solution pumped per unit time through the device. Typically, the dwell time will be less than about 5 seconds, preferably less than about 1 second and most preferably less than about 0.2 seconds. This feature of the continuous process such that the solutions are rapidly and fully admixed while being present in the mixing chamber for a minimal period ensures that initiation of microcrystal formation takes place in the same environment throughout the process. Constant initiation or nucleation conditions may lead to microcrystals with a more homogeneous size and shape and help minimise fusion together of microcrystals. Particles with a narrower distribution of size and shape are generally easier to process and are, for example, more suited to production of pharmaceutical formulations because it is easier to prepare unit doses.

The present inventors have surprisingly discovered that using a high flow-rate three-stream continuous mixer for the coprecipitation process it is possible to introduce a concentrated coprecipitant aqueous solution and a bioactive molecule solution into a mixing device via separate continuous streams. Within the mixing flow device these separate aqueous inputs are admixed with a third larger input of water miscible solvent and the coprecipitation initiated. A mixture may flow out of the mixing device and a resultant suspension of particles may be collected in a holding vessel or immediately processed further by, for example, concentrating, filtering or centrifuging followed by drying.

The successful application of a three stream process is surprising because it had been previously shown that simultaneous drop-wise addition of an aqueous protein solution and separate concentrated coprecipitant solution into a vessel containing excess of solvent, with rapid mixing, did not lead to protein coated microcrystals. Rather it resulted in the precipitation of two separate components i.e. protein aggregates and uncoated coprecipitant crystals. It was therefore believed the two components, protein and coprecipitant, needed to be intimately mixed prior to addition to a water-miscible solvent. Without wishing to be bound by theory it appears that the efficient mixing of the three input flows that can be achieved in the high flow-rate precipitator leads to near steady-state conditions such that nucleation of coprecipitant crystals by the bioactive molecules becomes competitive with self-nucleation leading to formation of coated crystals rather than the expected phase separation.

Surprisingly, the three stream process can be operated for extended periods with no blocking of inlet tubes as might be expected with such a co-precipitation process. Advantageously, the particles produced by the process are found to be highly consistent in size, shape and yield over the whole operating cycle indicating the co-precipitation conditions remain constant. A further advantage is that the flow system may run for many hours unattended and in so doing produce large quantities of particles.

Significant advantages arise because the aqueous solutions may be introduced separately into a mixer in a three-stream mixer device compared to a two-stream device where a pre-formed aqueous mixture needs to be introduced. Several of these advantages relate to the fact that each solution can be held under different conditions and have different compositions. For example, the bioactive molecule solution may be held at low temperatures such as less than 10 °C and can contain stabilisers such as surfactants or other additives at a higher concentration than may be possible if the solutions were combined. This is particularly so where the weight percent loading of protein on the microcrystals is required to be low. In this case for a three-stream process much less bioactive molecule solution needs to be introduced into a mixer than coprecipitant solution so the total amount of additives and stabilisers present during coprecipitation and in the final particles will be reduced.

Separating the first and second aqueous solutions also has the advantage that it makes screening for the best conditions for formation of particles of preferred morphology more efficient and minimises the amount of bioactive molecule required. For example, it is possible to vary the bioactive-molecule to coprecipitant ratio and the water-solvent ratio independently of each other. It has also been discovered that the three line system can be used to provide a very efficient factorial experimental design protocol that minimises the number of separate experiments and solutions required to optimise the system for production of particles. Thus, using fractional factorials it is possible to efficiently screen for coated microcrystals that have properties tailored for a specific application. In addition with the three-stream approach a bioactive molecule solution may be used directly as supplied from, for example, a supplier or a down-stream purification process. This has the advantage of minimising the amount of solution handling prior to coprecipitation and production of particles. Handling can increase the risk of loss of some of the bioactive molecule by, for example, aggregation or adsorption to vessels or filters.

Since the overall system may be sealed and sterilised and each of the three streams can be independently filtered through a sterile filter, the whole process may also be made sterile as required for pharmaceutical formulation manufacture.

Typically, the first aqueous solution comprising coprecipitant may be prepared as a substantially saturated or highly concentrated aqueous solution that is 30-250% of the equilibrium saturated concentration at the temperature prepared. If required, the first aqueous solution may be stored at a different temperature or pH and may contain different additives such as solvent from the second aqueous solution comprising bioactive molecules. Higher temperatures such as between about 30°C and about 95°C may be used to increase the concentration of the coprecipitant dissolved in the aqueous solution and addition of solvent can be used to either lower or increase the solubility as required. The initial concentration of coprecipitant affects the degree of super-saturation that may be achieved on mixing with the other solutions and this may affect the size of the microcrystals obtained. For smaller microcrystals that may, for example, be better suited for pulmonary formulations, higher initial concentrations are generally required. Using a concentration that is about 150 % of the room temperature saturation limit may lead to a reduction in particle size of greater than 20%. This leads to improved aerodynamic properties such as fine particle fraction. Preferably, the coprecipitant should be chosen to exhibit a substantially lower solubility in the third solution comprising water miscible solvent than in the aqueous solution.

The second aqueous solution comprising bioactive molecules may be prepared by starting from a solid preparation, for example, as a powder, which is to be dissolved in the aqueous solution. Alternatively, the bioactive molecule or mixture of molecules may be provided as a solution or suspension. The solution may also contain excipients that are suitable for stabilisation of the bioactive molecule in aqueous solution. Such excipients are well known in the art and may include surfactants, buffers, anti-oxidants and stabilisers. The pH of the second aqueous solution may be different from the first aqueous solution. The pH may for example be chosen to be closer to the pI of the bioactive molecule than in the first solution. Following preparation, the solution may be stored under conditions that minimise loss of bioactivity or integrity over the period prior to addition. This may require storage of the solution at a temperature different from those used for the other solutions such as for example less than 10 °C or at about 4 °C. The solution may also be stored under a sterile atmosphere, such as argon, nitrogen, neon and helium to prevent chemical degradation.

The first aqueous solution comprising coprecipitant molecules and the second aqueous solution comprising bioactive molecules may be admixed with a third solution comprising, for example, a substantially water miscible organic solvent or water miscible mixture of solvents, preferably one where the solvent or solvent mixture is substantially fully miscible with the coprecipitant solutions. Typically, the aqueous solutions are added to an excess of water miscible organic solvent. The excess of fully water miscible organic solvent is such that the final water content of the solvent/aqueous solution is generally less than about 30 vol%, typically less than about 10-20 vol% and conveniently less than about 8 vol%. In this manner, the organic solvent may preferably initially contain less than about 0.5-5 vol% water or be substantially dry, but may not necessarily be completely dry.

Any water miscible solvent that does not adversely affect the bioactive molecule may be used for the coprecipitation. Typical water miscible organic solvents may, for example, be short-chain alcohols such as: methanol; ethanol; propan-1-ol; propan-2-ol; aldehydes or ketones such as acetone, esters such as ethyl lactate, ethers such as tetrahydrofuran, diols such as 2-methyl-2,4- pentanediol, 1,5-pentane diol, and various size polyethylene glycol (PEGS) and polyols; or any combination or mixture thereof. Generally for preparation of pharmaceutical formulations the solvent should be non-toxic and generally regarded as safe (GRAS) such as a Class 3 solvent but for other applications solvents such as acetonitrile may be used.

In certain circumstances, the organic solvent may be pre-saturated with the bioactive molecule and/or coprecipitate to ensure that on addition of the aqueous solution the two components precipitate out together. This reduces the loss of material to the solvent and makes control of bioactive molecule loading per particle and hence doseage easier. The choice of solvent can lead to microcrystals with different sizes or shapes. It may also effect the efficiency of binding of protein to the crystals.

The coating may also comprise nanoparticles as adjuvants, or vectors or to alter the bioavailability of the bioactive molecule. The nanoparticles or their precursors may be advantageously included in the first aqueous solution and/or the third solvent to separate them from the bioactive molecules prior to coprecipitation. Suitable nanoparticles made from polymers, biomolecules and organic and inorganic materials are known in the art.

It should be understood that the term "admixed" refers to a process step wherein the water miscible organic solvent and aqueous solutions may be very rapidly combined or agitated together. Preferably, this should provide a near homogeneous mixture that is expelled from a mixing chamber before significant microcrystal growth can occur. Coprecipitants that crystallise extremely rapidly are therefore not suitable for use in a three-stream continuous flow mixing device because crystal growth may be significant within the mixing chamber. This may favour particle fusion and/or separate precipitation of the coprecipitant and the bioactive molecule. Rapidly crystallising coprecipitants may, for example, be ionic salts.

Typically, the admixing of the first aqueous solution comprising coprecipitant molecules and the second aqueous solution comprising bioactive molecules may occur in a process wherein the continuous aqueous streams of bioactive molecules and coprecipitant are mixed together with a much larger volume of third solution comprising water miscible organic solvent. The combined mixture flowing out of the mixing device typically contains more than about 75 vol% solvent, preferably more than about 85 vol% solvent and most preferably between about 90 and about 99.5 vol% solvent. The water miscible solvent stream may contain water at less than about 5 vol% and/or be substantially saturated with coprecipitant to aid coprecipitation.

Advantageously, the microcrystals may be collected as a suspension in a solvent using a holding vessel held at various pressures including atmospheric pressure.

Running a continuous process under conditions close to ambient may lead to reduced capital and operating costs relative to conventional methods of forming particles for pharmaceutical applications such as spray-drying or super-critical fluid processing. It is envisaged that large quantities of bioactive molecule coated particles, for example, may be produced in this manner on an industrial scale.

In the continuous co-precipitation method a first pump may continuously deliver the first aqueous solution comprising coprecipitant molecules, a second pump may continuously deliver the second aqueous solution comprising bioactive molecules and a third pump may continuously deliver the third solution comprising water miscible solvent. Further pumps may be used if an additional component such as a coating or cross-linking compound or polymer requires to be introduced. It may be necessary to introduce such solutions via a second mixing device linked in series with the first so that the formation of microcrystals is not disturbed. The length of tubing may be altered to ensure microcrystal formation is either completed or interrupted. Coating or cross-linking can be used to alter the solubility, bioactivity and bio-availability exhibited by the microcrystals. Alternatively, the bioactive molecules solution may be mixed with excess solvent in the first mixing device and then this mixture pumped into a second mixing device and admixed with coprecipitant or else the coprecipitant solution may may be mixed with excess solvent in the first mixing device and then this mixture pumped into a second mixing device and admixed with the bioactive molecule solution. These alternate mixing modes may be used to control the size of the particles and /or efficiency of binding of the bioactive molecule to the formed microcrystals. The pumps may be of any suitable type but must accurately and precisely deliver the solutions at a defined flow rate and be compatible with the bioactive molecules employed. Conveniently, HPLC pumps or the like can be used since these are optimised for delivering aqueous solutions and water miscible solvents over a range of flow rates. Preferably, the pumps exhibit near pulse-less operation to ensure the relative flow rates of the two aqueous solutions remain constant as this will affect the loading. Alternatively peristaltic or centrifugal type pumps may be used since these can also be used to deliver aqueous and solvent streams accurately and may have the advantage of reduced cost and ease of cleaning. Because of difference in flow rates, cleaning and solvent compatibility it may be advantageous to use different types of pump to deliver the different inlet streams.

Typically, the aqueous solutions may be delivered at flow rates between about 0.1 ml/min and about 1000 ml/min. For screening applications, the aqueous solutions will preferably be delivered at flow rates between about 0.2 ml/min and about 20 ml/min and for manufacture the aqueous solutions will typically be delivered between about 2 ml/min and about 1000 ml/min or preferably between about 5 ml/min and about 1000 ml/min. The aqueous pump heads and lines may be made of material that resists fouling by the bioactive molecule. The water miscible solvent may generally be delivered about 4-100 times faster than the aqueous solutions and less precision is required so a more powerful/efficient pump such as a centrifugal pump may be required. Typically, the solvent may be delivered at about between about 2 ml/min and about 20,000 ml/min and for screening applications preferably between about 2 ml/min and about 200 ml/min. For manufacture the solvent may preferably be delivered between about 20 ml/min and about 20,000 ml/min and more preferably between about 500 ml/min and about 20,000 ml/min.

A mixing device may provide a method for rapidly and intimately admixing two continuous aqueous streams of the first and second aqueous solutions with a continuous stream of the third solution comprising water miscible solvent stream such that precipitation begins to occur almost immediately.

The mixing device may be any device that achieves rapid mixing of the three solutions. Thus it can, for example, be a static device that operates by shaping/combining or impinging the incoming liquid flow patterns such as via a coaxial, T-shape , Y-shape or more complicated geometry or else a dynamic device that actively agitates the three fluid streams together or else a combination. In a dynamic device, agitation of the three streams may be achieved by use of a variety of means such as stirring, sonication, shaking or the like. Methods of stirring include a paddle stirrer, a screw and a magnetic stirrer. If magnetic stirring is used a variety of stirring bars can be used with different profiles such as, for example, a simple rod or a Maltese cross. The material lining the interior of the mixing device may preferably be chosen to prevent significant binding of the bioactive molecule or the particles onto it. Suitable materials may include medical stainless steel, titanium, glass, silicone and Teflon (Registered Trade Mark).

Alternatively, the mixing device may allow either the first or second aqueous solutions to be mixed with the third solution, and thereafter the remaining of either the first and second aqueous solutions to be added.

Depending on the production scale required the mixing device may be produced in different sizes and geometries. The size of the mixing chamber required is a function of the rate of flow of the solvent streams. For flow rates of about 0.025 - 10 ml/min of aqueous solution and about 2.5 - 200 ml/min of solvent it is convenient to use about a 0.2 ml mixing chamber. For higher flow rates a proportionally larger mixing chamber or device such as about 1.0 - 10.0 ml may be preferred.

Typically, in a continuous process the bioactive molecule and coprecipitate solutions are added to an excess of water miscible organic solvent. This entails a smaller volume of aqueous solutions being added to a larger volume of the excess of organic solvent such that rapid dilution of water from the bioactive molecule/coprecipitate solution into the organic solvent occurs with an accompanying rapid dehydration of the bioactive molecule and formation of microcrystals.

The temperature at which the precipitation may be carried out may be varied. For example, the aqueous solutions and the solvent may be either heated or cooled. Cooling of the bioactive molecule solution and organic solvent may be useful where the bioactive molecule is fragile. Alternatively, the solvent and aqueous mixtures may all be at different temperatures. For example, it may be preferable to heat the coprecipitant solution so that a high coprecipitant concentration can be achieved while keeping the solvent at room temperature and the bioactive molecule at reduced temperature such as less than about 10 °C. If necessary the solvent may be held at a temperature below the freezing point of the aqueous mixture. Moreover, the pressure may also be varied, for example, higher pressures may be useful to reduce the volatility of the solvent.

Upon admixing the bioactive molecule/coprecipitant solutions to the excess of the water miscible organic solvent, coprecipitation of the bioactive and coprecipitant occurs rapidly. Typically, following a priming period of around about 1 to 5 minutes the mixture exiting the mixer may become slightly opaque due to the presence of microcrystals less than about 30 seconds after combining the three streams and most typically after less than about 10 seconds. If necessary a seeding process may be used to prime the mixing device. For example the flows may be temporarily halted and then restarted.

The precipitated particles may, if required, be further dehydrated by rinsing with fresh organic solvent that may be dry or contain low amounts of water. Preferably solvent with a water content of 0.5-8% is used. Within this range the bioactive molecule often advantageously retain higher bioactivity. Rinsing may also be useful to remove residual solvent saturated in coprecipitant. On drying this residual coprecipitant may otherwise serve to cement particles together leading to the formation of aggregates. Rinsing with solutions of excipients prior to drying or storage may also be used to introduce other excipients onto the particles.

It has advantageously been found that the precipitated microcrystals may be stored in an organic solvent and that the bioactive molecules display extremely good retention of activity and stability over an extended period of time. Moreover, precipitated bioactive molecules stored in an organic solvent, will typically be resistant to attack by bacteria, thus increasing their storage lifetime.

With time the coprecipitate will settle, which allows easy recovery of a concentrated suspension of microcrystals by decanting off excess solvent. The copecipitate may, however, be subjected to, for example, centrifugation and/or filtration in order to more rapidly recover the precipitated particles. Conventional drying procedures known in the art such as air drying, vacuum drying or fluidised bed drying may be used to evaporate any residual solvent to leave solvent free microcrystals.

Alternatively, solvent may be removed from the microcrystals in a drying procedure using compressed gasses such as hydrofluorocarbons like HFA-134a or supercritical fluids such as supercritical $CO_2$. Typically, microcrystals in a solvent prepared in a three stream continuous process, may be loaded into a high pressure chamber with compressed gas or supercritical fluid such as $CO_2$ flowing through the suspension until the solvent (or as much as possible) has been removed. Compressed gases of hydrofluorocarbons such as HFA-134a or HFA-227 will dissolve the coprecipitant solvent and can be flowed through until the concentration has reached the required level. The pressure may then be released to provide a dry powder or else the particles left in HFA as a suspension. Suspensions of bioactive coated microcrystals in hydrofluorocarbons may be suitable for multi-dose inhaler devices (MDI). This technique removes virtually all residual solvent from the microcrystals. This is of particular benefit for pharmaceutical formulation since residual solvent may lead to unexpected physiological effects. A further advantage of compressed gas or super-critical fluid drying of the suspensions is that it can be used to produce powders and pharmaceutical formulations with much lower bulk density than obtained by other isolation techniques. Typically bulk densities lower than about 0.1 g/ml may be obtained. Low bulk density formulations are particularly useful for pulmonary delivery of bioactive molecules since they generally contain fewer strongly bound aggregates. The critical point drying or compressed gas extraction may be carried out in a number of different ways known in the art.

It is therefore possible to set up a continuous co-precipitation system to form microcrystals according to the first aspect and, in fact, any other type of particles and then dry the particles using compressed gases or supercritical fluids such as supercitical $CO_2$.

For pharmaceutical applications, dry precipitated particles (i.e. microcrystals) may be typically introduced into a sterile delivery device or vial under sterile conditions prior to use. Alternatively, the particles may be transferred into the sterile delivery device or vial as a suspension in solvent under sterile conditions. They may then be optionally dried in situ using for example HFA or supercritical $CO_2$ drying.

The methods described herein may also allow organic soluble components present in the aqueous solution to be separated from the bioactive molecules. Typical components that may need to be removed include buffers, surfactants, hydrophobic biomolecules such as lipids or lipoproteins and denaturants. For example, a buffer such as Tris which in its free base form is soluble in an organic solvent like ethanol may be separated from the bioactive molecule during precipitation. However, it may be necessary to convert the buffer to the free base by the addition of another organic soluble base to the aqueous solution or organic solvent. Thus the present invention also discloses an efficient continuous method of removing undesirable components from the bioactive molecule such that the undesirable components are not coprecipitated with the bioactive molecule and so remain dissolved in the organic phase. This may be achieved by the inclusion of additives such as acids, bases, ion-pairing and chelating agents in aqueous or organic solvent prior to bioactive molecule precipitation of the non-hygroscopic coated particles. The bioactive molecules may therefore be coated in a highly pure form.

The formulations described in the invention may typically be produced at a number of dosage strengths. The dosage may be conveniently varied by varying the percentage weight of bioactive molecule per particle from below about 0.1 wt% up to about 50 wt%. A particular advantage of using a three-stream continuous flow precipitator is that it is possible to easily vary the loading of bioactive molecule. This is particularly so for bioactive molecules that may have high solubility in a normal buffered aqueous solution but are poorly soluble in the presence of the coprecipitant. For example, it is possible to introduce a concentrated solution of a coprecipitant into a mixer at a slower rate than that of the bioactive molecule such that higher loadings are achievable than starting with a mixture. The carrier solubility may provide the possibility of producing particles that contain bioactive molecules at loadings from about 50 wt% to less than about 1 wt% or even less than about <0.1 wt% so that the dosage strength of the pharmaceutical formulation can be conveniently varied. The carrier solubility in aqueous solution at room temperature may range from about 2-200 mg/ml and more preferably be in the range about 10-150 mg/ml.

High loading bioactive molecule coated microcrystals provide a convenient method for concentrating a bioactive molecule. Thus, microcrystals made with a low solubility coprecipitant may be suspended at high concentration in aqueous solution at levels such that the solubility limit of the coprecipitant is significantly surpassed. The bioactive molecule on the surface may continue to dissolve into aqueous to produce a concentrated solution that may be easily separated from the remaining core microcrystals. Glutamine is a preferred coprecipitant for this process because of it's low solubility in aqueous solution. For example, if around 20 wt% loading glutamine microcrystals are prepared using a 5 mg/ml bioactive molecule solution, then suspension of about 125 mg of these crystals in an aqueous solution may generate approximately a 25 mg/ml of bioactive molecule in a saturated glutamine solution, corresponding to about a 5 fold concentration step. Using this type of process it is possible to obtain around about a 2-20 fold increase in concentration of a bioactive molecule. This may provide cost or time advantages over other concentration techniques such as ultrafiltration or freeze-drying

With a high flow rate mixer coprecipitant concentrations of less than about 160 mg/ml or preferably less than 80 mg/ml can advantageously be used to produce pharmaceutical formulations containing free-flowing particles. The particles typically have a narrow size distribution with a mean particle size of less than about 50 microns. Higher concentration may be used at higher temperature. Formulations containing a narrow size distribution of coated crystals provide improved delivery reproducibility and hence better clinical performance.

The pharmaceutical formulations described can be conveniently produced in a sterile form by pre-filtering the aqueous and organic solutions through about 0.2 micron filters prior to admixing them in a contained sterile environment. Pharmaceutical formulations should be substantially free of harmful residual solvents and the present invention typically provides powders containing less than about 0.5 wt% of a Class 3 solvent following conventional drying procedures. Substantially lower solvent levels are obtainable by vacuum or air drying and by flowing compressed gasses or super-critical fluids such as $CO_2$ through a suspension of the microcrystals in a dry water miscible and $CO_2$ miscible solvent.

The present method may also be used to make bioactive molecule coated microcrystals suitable for pharmaceutical formulations using water-soluble bioactive compounds that are much smaller than typical biological macromolecules. These formulations may be made either by a batch or a continuous process and may advantageously employ a non-hygroscopic carrier such as D,L-valine. Water-soluble bioactive drugs including antibiotics based on aminoglycosides such as tobramycin sulphate, amikacin, gentamycin and kanomycin or antimicrobial peptides such as novispirin, and plectasin may be used. Preferably, the bioactive molecules may be polar and contain one or more functional groups that may be ionised at the pH used for coprecipitation. The bioactive molecule may also preferably have a largest dimension greater than that of the unit cell formed by the core material on crystallisation. This will favour formation of bioactive molecule coated microcrystals and minimise the possibility of inclusion of the bioactive molecule within the crystal lattice.

It is clear from the description above that many different parameters can be altered that potentially alter the physical properties of the coated microcrystals and also the bioactivity and bioavailability of the bioactive molecules.

According to a second aspect of the present invention there is provided a pharmaceutical formulation comprising particles that contain one or more microcrystals wherein the microcrystals comprise:

    (a) a substantially non-hygroscopic inner crystalline core formed from coprecipitant molecules; and
    (b) an outer coating comprising one or more bioactive molecules;

wherein the coated microcrystals have been formed in a single continuous process by mixing a first aqueous solution comprising coprecipitant molecules, a second aqueous solution comprising bioactive molecules and a third solution comprising water miscible solvent substantially simultaneously; or mixing either the first and second aqueous solutions with the third solution and thereafter mixing with the remaining of either the first and second aqueous solutions.

By substantially non-hygroscopic herein is meant that the crystalline core does not readily take-up and retain moisture. Typically, the particles will not aggregate nor will the core under-go significant changes in morphology or crystallinity on exposure to greater than about 50% relative humidity and preferably greater about 80% relative humidity at room temperature.

By crystalline core is meant that the constituent molecules or ions are organised into a solid 3-dimensional crystal lattice of repeating symmetry that can be identified by powder x-ray diffraction. Typically, a well-defined melting endotherm (i.e. not a glass transition) may be observed on heating the particles in a differential scanning calorimeter (DSC). This is a well-known characteristic showing crystallinity and also shows that the crystalline core may be generally substantially composed of solid-state phases that are thermodynamically stable at room temperature and ambient humidity.

By continuous process is meant that the molecules that provide the crystalline core and the bioactive molecules that provide the outer coating, precipitate out of solution together directly in the form of coated particles. This is evidently different from a template approach in which pre-formed crystals are treated and then exposed to a coating material. The single step process means there is no requirement for a separate coating or milling step. It should also be understood that particle formation does not require any evaporative processes such as occur for example in spray-drying or freeze-drying. It also does not require supercritical fluids with particle formation taking place within a mixture of fully miscible conventional solvents of substantially similar densities.

The particles may be used in a medical application such as a therapy or a diagnostic method such as in a kit form to detect, for example, the presence of a disease. Diseases which may include diseases of the lung such as lung cancer, pneumonia, bronchitis and the like, where the particles may be delivered to the lung and the lung capacity/effectiveness tested, or disease causing agents identified. The particles may be used in veterinary uses.

Typically, the coating of bioactive molecules may be substantially continuous. Alternatively, it may be advantageous to have a pharmaceutical formulation comprising particles with a substantially discontinuous coating of bioactive molecules. The coating may also vary in thickness and may range from about 0.01 to 1000 microns, about 1 to 100 microns, about 5 to 50 microns or about 10 to 20 microns. The coating of bioactive molecules may be continuous or discontinuous.

The pharmaceutical formulation may desirably comprise particles with a narrow size distribution. Typically, the pharmaceutical formulation may therefore comprise a substantially homogeneous system with a significant number of particles having generally the same or similar size.

Microcrystals and bioactive molecule coated microcrystals produced by a continuous process typically exhibit a narrow size distribution as assessed by dynamic laser light scattering with a Span less than 2, preferably less than 1.8 and more preferably less than 1.5. Bioactive molecule coated microcrystals produced by coprecipitation are typically advantageously smaller than microcrystals produced by precipitation of the pure carrier material. This is consistent with coating of the bioactive molecule on the microcrystal surface. Span values are calculated as follows:

d(0.1) ($\mu$m) = 10% of the particles are below this particle size.
d(0.5) ($\mu$m) = 50% of the particles are above and below this particle size.
d(0.9) ($\mu$m) = 90% of the particles are below this particle size.
Span = d(0.9) - d(0.1) / d(0.5).

The particles may have a maximum cross-sectional dimension of less than about 80$\mu$m, preferably less than about 50$\mu$m across or more preferably less than about 20$\mu$m. By maximal cross-sectional dimension is meant the largest distance measurable between the diametrically opposite points.

The molecules making up the crystalline core may typically each have a molecular weight less than about 2kDa. Preferably, the molecules making up the crystalline core each have a molecular weight of less than about 1kDa. More preferably, the molecules making up the crystalline core each have a molecular weight of less than about 500 Daltons. Preferred molecules are those that can be rapidly nucleated to form crystals on undergoing precipitation. Molecules that provide particles that consist substantially of amorphous aggregates or glasses are therefore generally not suitable as core materials.

Typically, the molecules forming the crystalline core have a solubility in water at ambient temperature of less than about 150 mg/ml and preferably less than about 80 mg/ml. Surprisingly, it has been found by the present inventors that molecules with solubilities less than these values tend to produce crystals with improved flow properties. Free-flowing particles are generally preferred for many pharmaceutical manufacturing processes since they, for example, facilitate filling capsules with precise dosages and can be conveniently used for further manipulation such as coating. Free flowing particles are generally of regular size and dimensions, with low static charge. Needle shaped crystals of high aspect ratio are, for example, generally not free flowing and are therefore not preferred in certain formulations.

The molecules which make up the crystalline core may, for example, be: amino acids, zwitterions, peptides, sugars, buffer components, water soluble drugs, organic and inorganic salts, compounds that form strongly hydrogen bonded lattices or derivatives or any combinations thereof. Typically, the molecules are chosen so as to minimise adverse physiological responses following administration to a recipient.

Amino acids suitable for forming the crystalline core may be natural or unnatural and in the form of pure enantiomers or racemates. Examples include: alanine, arginine, asparagine, glycine, glutamine, histidine, lysine, leucine, isoleucine, norleucine, D-valine, L-valine, mixtures of D,L-valine, methionine, phenylalanine, proline and serine, taurine or any combination thereof. In particular, L-glutamine, L-histidine, L-serine, L-methionine, L-isoleucine, taurine, L-valine or D,L-valine are preferred. For amino-acids that have side-chains that substantially ionise under coprecipitation conditions it is preferable to use counterions that generate crystalline salts with low solubility and which are non-hygroscopic. Examples of other molecules and salts for forming the crystalline core may include, but are not limited to $\alpha$-lactose, $\beta$-lactose, mannitol, ammonium bicarbonate, sodium glutamate, arginine phosphate and betaines.

Typically, the molecules forming the crystalline core have a low solubility in water of, for example, between about 12-150 mg/ml and preferably about 20-80 mg/ml at about 25°C. Molecules with a solubility of above about 150 mg/ml in water may also be used to obtain free flowing particles provided that they are coprecipitated from a sub-saturated aqueous solution. Preferably they are coprecipitated at a concentration of about 150 mg/ml or less and more preferably of about 80 mg/ml or less. For molecules of high aqueous solubility at 25°C it may also be advantageous to use lower coprecipitation temperatures such as about 10°C or about 4°C so that they are closer to saturation at concentrations of 150 mg/ml or less. Similarly higher temperatures such as 35°C or 50 °C may be used for coprecipitation of core forming molecules poorly soluble at 25°C.

The molecules forming the crystalline core have a melting point of greater than about 90°C such as above about 120°C and preferably above about 150°C. Having a high melting point means that that the crystals formed have a high lattice energy. A high lattice energy increases the likelihood of the particles formed having a crystalline core with the bioactive molecule coated on the surface and will tend to minimise the amorphous content of the particles. Particles which contain amorphous material can undergo undesirable changes in physical properties on exposure to high humidities or temperatures and this can lead to changes in bioactivity and solubility which are undesirable for pharmaceutical formulation. It is therefore advantageous to use coprecipitant that results in particles with a high melting point since these will tend to form more stable pharmaceutical formulations. High melting point coprecipitants generally also have reduced aqueous solubility and so may be more suitable for coprecipitation within the preferred concentration range.

Coprecipitants that crystallise extremely rapidly such as for example inorganic salts such as potassium sulphate may not be suitable for use in a 3 line process.

Conveniently, bioactive molecules forming a coating on the crystalline core may be selected from any molecule capable of producing a therapeutic effect such as for example an active pharmaceutical ingredient (API) or diagnostic effect. By therapeutic effect is meant any effect which cures, alleviates, removes or lessens the symptoms of, or prevents or reduces the possibility of contracting any disorder or malfunction of the human or animal body and therefore encompasses prophylactic effects.

The coating of bioactive molecules may also comprise excipients commonly used in pharmaceutical formulations such as stabilizers, surfactants, isotonicity modifiers and pH/buffering agents. In the case of vaccines, the bioactive molecules coating may also include cross-linker molecules and adjuvants that are present to reduce the aqueous solubility and enhance the immune response.

The bioactive molecules may, for example, be: any drug, peptide, polypeptide, protein, nucleic acid, sugar, vaccine component, or any derivative or conjugate thereof or any combination which produces a therapeutic effect.

Examples of bioactive molecules include, but are not limited to drugs such as: anti-inflammatories, anticancer, anti-psychotic, anti-bacterial, anti-fungal; natural or unnatural peptides; proteins such as insulin, $\alpha$1 - antitrypsin, $\alpha$ - chymotrypsin, albumin, interferons, antibodies, fusion proteins such as Fc-fusion proteins, protein-drug conjugates; nucleic acids such as fragments of genes, plasmids, DNA, RNA or RNAi either from natural sources or synthetic such as oligonucleotides and antisense nucleotides; sugars such as any mono-, di- or polysaccharides; plasmids; protein/peptide-drug conjugates e.g. antibody fragments (such as Fc fragments-drug conjugates). A suitable drug may, for example, be an active agent against tuberculosis such as capreomcin.

The bioactive molecules of the present invention may also be in the form of a protein-peptide-drug conjugate, in which a drug or other suitably active biomolecule, is conjugated to a protein/peptide by way of suitable bonding, such as covalent bonding through, for example, reactive groups on the protein/peptide and the drug. A person skilled in the art is well aware of techniques known in the art to synthesise such conjugates. The protein/peptide components of such conjugates may be designed to facilitate entry into cells and may be, for example, TAT or Charriot, known in the art. Other peptides which facilitate entry into cells are disclosed in, for example, WO 01/41811, US 5,807,746 and WO 99/05302 which are incorporated herein by reference. Particularly preferred peptides for facilitating active uptake in the central region of the lung may be based on antibody molecules, especially Fc fragments of IgG, or other antibody fragments, which have been shown to be actively transported in the central lung. Similarly, bioactive molecules conjugated to nanoparticles may be used in a similar way.

Nucleic acids may for example be capable of being expressed once introduced into a recipient. The nucleic acid may thus include appropriate regulatory control elements (e.g. promoters, enhancers, terminators etc) for controlling expression of the nucleic acid. The bioactive molecule may also be a chemically modified derivative of a natural or synthetic therapeutic agent such as a PEG-protein.

The nucleic acid may be comprised within a vector such as a plasmid, phagemid or virus vector. Any suitable vector known to a man skilled in the art may be used.

Vaccine coating components may, for example, include antigenic components of a disease causing agent, for example a bacterium or virus, such as diptheria toxoid and/or tetanus toxoid. A particular advantage of such vaccine formulations is that they generally show greatly enhanced stability on exposure to high temperature when compared with conventional liquid preparations. Such formulations prepared according to the present invention can, for example, be exposed to temperatures of greater than 45°C for 48 hours and retain their ability to illicit an immune response when tested *in vivo,* whereas standard liquid samples are generally found to be completely inactivated. Vaccines that exhibit high temperature stability do not need to be refrigerated and therefore provide considerable cost savings in terms of storage and ease of distribution particularly in developing countries. Vaccines are useful for the prevention and/or treatment of infections caused by pathogenic microorganisms, including viral, fungal, protozoal, amoebic and bacterial infections and the like. Examples of vaccine formulations that can be prepared according to the present invention include sub-unit, attenuated or inactivated organism vaccines including, but not limited to, diphtheria, tetanus, plague, anthrax polio, pertussus and hepatitis A, B and C, HIV, rabies and influenza.

Formulations of vaccine coated microcrystals such as diphtheria taxoid coated D,L-valine or L-glutamine crystals may be stored under a range of different conditions and following reconstitution and inoculation may be found to illicit strong primary and secondary immune responses. Alternatively vaccine coated crystals may be formulated as dry powders for delivery to a recipient by a number of other routes including parenteral, pulmonary and nasal administration. Pulmonary delivery may be particularly efficacious for very young children.

Particles according to the present invention are also applicable to administration of polysaccharides linked to proteins such as HiB (haemopholis influenza B) and pneumococcal vaccines and live virus vaccines, such as mumps, measles and rubella. Particles according to the present invention may also be prepared with modern flu vaccine components such as MV A vectored influenza vaccine.

In addition vaccine component coated micro-crystals may be useful for formulation of vaccines developed for cancers, especially human cancers, including melanomas; a skin cancer; lung cancer; breast cancer; colon cancer and other cancers. Pulmonary formulations as described herein may be particularly suited for treatment of lung cancer. It should be noted that in addition to protein based vaccines (i.e. protein/peptide components coated on an inner substantially non-hygroscopic crystalline core) nucleic acid based vaccine formulations may also be prepared according to the present invention, wherein nucleic acid molecules are coated on an inner substantially non-hygroscopic crystalline core.

Examples of non-hygroscopic coated particles which have been found to have advantageous properties include those with a crystalline core of D,L-valine and a coating of insulin; a crystalline core of L-glycine and a coating of antitrypsin, a crystalline core of Na glutamate and a coating of insulin; a crystalline core of L-methionine and a coating of insulin; a crystalline core of L-alanine and a coating of insulin; a crystalline core of L-valine and a coating of insulin; a crystalline core of L-histidine and a coating of insulin; a crystalline core of L-glycine and a coating of $\alpha$ - antitrypsin; a crystalline core of L-glutamine and a coating of albumin: a crystalline core of D,L-valine and a coating of oligonucleotides DQA-HEX; a crystalline core of D,L-valine and a coating of $\alpha$1-antitrypsin with a further anti-oxidant outer coating of N-acetyl cystein; a crystalline core of D,L-valine and a coating of ovalbumin; a crystalline core of L-glutamine and a coating of ovalbumin, a crystalline core of D,L-valine and a coating of diptheria taxoid; a crystalline core of L-glutamine and a coating of diptheria taxoid; a crystalline core of D,L-valine and a coating of diptheria taxoid; a crystalline core of the L-glutamine and a coating of tetanus taxoid; a crystalline core of the D,L-valine and a coating of a mixture of diptheria taxoid and tetanus taxoid; a crystalline core of L-glutamine and a coating of a mixture of diptheria taxoid and tetanus taxoid.

Typically a batch of particles formed under well controlled conditions is composed of individual microcrystals that all exhibit substantially the same morphology or crystal-shape and which have a narrow size distribution. This can be conveniently observed in SEM images and verified by particle size measurements. The microcrystals according to the present invention typically have a maximum cross-sectional dimension and largest dimension of less than about 80 microns. Preferably they have a maximum cross-sectional dimension of less than about 40 microns and more preferably less than 20 microns. Particles with a maximum cross-sectional dimension of between about 0.5 and about 20 micron are most preferred. Alternatively free-flowing powders of spherical aggregates of similar sized microcrystals may be formed with maximum cross-sectional dimension of less than about 50 microns and preferably less than about 20 microns. A notable aspect of the particles formed with preferred coprecipitants is that their size and morphology remain substantially constant on exposure to high humidities such as up to about 80 % RH. In addition their free-flowing characteristics and aerodynamic properties may be retained on re-drying.

The amount of bioactive molecule coated onto each particle can be conveniently varied by changing the ratio of bioactive molecule to core molecule in the initial aqueous solution prior to coprecipitation. Typically the bioactive molecule will make up between about 0.1 wt% and about 50 wt% of each coated microcrystal. More preferably the loading of bioactive molecule in the particles will be between about 1 wt% and about 40 wt%.

Typically, at least some of the bioactive molecules retain a high level of activity even after exposure to high humidity.

Typically, the non-hygroscopic coated particles are stable (i.e. substantially retain their bio-activity) on exposure to elevated temperatures and may be stable at up to about 60°C for more than 1 week. This aids the storage and shows pharmaceutical formulations formed from the non-hygroscopic coated particles may be expected to have extended shelf-lives even under non-refrigerated conditions.

Typically, the core material of the non-hygroscopic coated particles will absorb less than about 5 wt% of water and preferably less than about 0.5 wt% at relative humidities of up to about 80%. Particles comprising biomolecules will typically absorb higher amounts of water with the wt% depending on the loading

Typically, the bioactive molecules coated on the crystalline core retain a native or near-native configuration i.e. the bioactive molecules are not irreversibly denatured during the production process. Coating of the bioactive molecules onto the crystalline core is also advantageously found to lead to enhanced stability on storage of the particles at ambient or elevated temperatures. For example, typically the bioactive molecule may retain most of its bioactivity when reconstituted in aqueous media. Preferably the bioactive molecule will retain greater than about 50% of it's initial bioactivity after storage at 25°C for 6 months. More preferably the bioactive molecule will retain greater than about 80% of its bioactivity and most preferably greater than about 95% bioactivity.

The fine free-flowing particles or suspensions described typically do not adhere to the walls of a glass vial. The particles typically re-dissolve rapidly and completely in water, aqueous solutions (containing buffers and salts such as those commonly used for reconstitution) or else in physiological fluids. Full redissolution of a dry powder or suspension will generally take place in less than about 2 minutes, preferably in less than about 60 seconds and most preferably in less than about 30 seconds. Formulations reconstituted in aqueous buffer are typically low turbidity, colourless solutions with clarity better than about 15 FNU and preferably better than about 6 FNU (FNU = Formazine nephelometric units). Alternatively suspension of the microcrystals in a saturated aqueous solution of the parent coprecipitant can be used to slow dissolution rates.

Commonly bioactive molecules require excipients or stabilising agents to be present when dissolved in aqueous solution such as buffer compounds, salts, sugars, surfactants, anti-aggregants and antioxidants. These may be included in the starting aqueous solution and incorporated into the particles during the coprecipitation process. They will then be present on reconstitution of the particles for example as a pharmaceutical formulation. Typically following coprecipitation of all the components the excipients will be concentrated on the outer surface of the particle and will permeate into the coating of bioactive molecules. A typical antioxidant may, for example, be cysteine such as in the form of N-acetyl cysteine while a typical surfactant may be Tween. During coprecipitation it is possible for the relative ratio of excipients to bioactive molecule to change due to dissolution into the solvent. This may be controlled by pre-addition of selected excipients to either the initial aqueous solution, the coprecipitation solvent or the rinse solvent such that on drying the desired ratio is obtained in the particles. Thus, for example, organic soluble sugars or polymers may be coated onto the surface of protein coated particles by inclusion in the rinse solvent in order to provide enhanced storage stability. Alternatively additives such as salts or nanoparticles may be included in the coprecipitation or rinse solvent and coated onto the outer surface of the particles in order to improve the physical or therapeutic properties of the particles. For example it is found to be advantageous to provide isoleucine coated insulin-glycine particles by rinsing the formed microcrystals with a solution of isoleucine in 2-propanol prior to drying. These particles have enhanced flow and aerodynamic properties relative to the uncoated ones.

According to a third aspect of the present invention there is provided a pharmaceutical formulation for pulmonary delivery comprising microcrystals formed according to the first aspect.

In order to use inhalation to administer drug molecules into the bloodstream, the drug must be made into a formulation capable of being delivered to the central airways or else the deep lung depending on the application. In the deep lung the bioactive molecule may be transported passively into the blood stream and the efficiency will tend to be lower for larger molecules. In regions of the lung where active transport can occur, for example by receptor mediated transport, then it may be possible to more efficiently deliver larger bioactive molecules into the blood stream. This active transport pathway may be preferred for bioactive molecules with a molecular mass greater than about 50 kDa and more preferably for molecules with a molecular mass greater than about 100 kDA For example, bioactive molecule coated microcrystals comprising antibodies with an Fc-region such as IgG or Fc-conjugates such as Fc-fusion proteins or Fc-drugs may be used for delivery to the central airways. This may be via FcRn mediated transport or via other receptors that for example specifically transport other molecules such as albumin. In the case of dry-powder for delivery to the deep lung, this generally requires particles with mass median dimensions in the range of about 1-5 microns, although it has been demonstrated that larger particles with special aerodynamic properties may be used. For delivery to the central airways larger particles are preferred with mass median aerodynamic diameters in the range of about 3-30 microns. These can advantageously be produced in a 3 line coprecipitation process by tuning the coprecipitation conditions using Design of Experiment techniques as described below. It is recognised that because the particles disclosed are generally non-spherical the mass median aerodynamic diameter may be smaller than the mass median geometric diameter. Certain formulations of particles according to the present invention may be suitable for forming pulmonary formulations as they can be used to generate fine free-flowing particles well suited to delivery by inhalation. Given that the bioactive molecule is on the surface of these non-hygroscopic coated particles, the particles generally exhibit unexpectedly low static charge and are straightforward to handle and use in a delivery device as a dry powder. Alternatively, for example, they can be used as a suspension in a nebulisor.

In particular, bioactive molecules suitable for the formation of pulmonary pharmaceutical formulations may include but are not restricted to any of the following: therapeutic proteins such as insulin, $\alpha$1-antitrypsin, interferons; antibodies and antibody fragments and derivatives; therapeutic peptides and hormones; synthetic and natural DNA including DNA based medicines; enzymes; vaccine components; antibiotics; pain-killers; water-soluble drugs; water-sensitive drugs; lipids and surfactants; polysaccharides; or any combination or derivatives thereof. The pulmonary formulation comprising particles may be used directly in an inhaler device to provide high emitted doses and high fine particle fractions. Thus emitted doses measured in a MSLI (stages 1-5) are typically greater than about 70% and preferably greater than 90%. The fine particle fractions measured in a MSLI (stages 3-5) are typically greater than about 20% and preferably greater than about 40% and more preferably greater than about 55%. The fine particle fraction is commonly defined as the fraction collected on the lower stages of a multi-stage liquid impinger (MSLI) and corresponds to particles with aerodynamic properties suitable for administration to the deep lung by inhalation i.e. less than about 3.3 microns. Pulmonary formulations may also be prepared that target the central airways. These will also have similarly high emitted doses but will tend to be collected in stages 1-2 of an MLSI with low FPF. These may advantageously be made with regulatory authority approved excipients such as lactose. The pulmonary formulation may be used in a dry powder delivery device without any further formulation with, for example, larger carrier particles such as lactose.

For pulmonary formulations, targeted to the deep lung, particles with a mass median aerodynamic diameter less than about 10 microns and more preferably less than about 5 microns are preferred. For delivery to the the central airways particle with a mass median aerodynamic diameter in the range of about 4-20 microns and more preferably in the range of about 5-10 microns are preferred. These will typically have a mass median diameter similar to their mass median aerodynamic diameter. Typically, free-flowing, non-hygroscopic low static particles with maximum cross-sectional diameters in the range of about 1-5 microns for the deep lung or 5-10 microns for the central airways are preferred. These can be obtained using amino-acids such as for example, L-glutamine to form the crystalline core. However, the inventors have surprisingly discovered that bioactive molecule coated microcrystals that take the form of high aspect ratio flakes may advantageously have mass median aerodynamic diameters smaller then their maximum cross-sectional diameters. Suitable shapes may be, for example, leaf shaped or tile shaped. With such particles the preferred range of maximum cross-sectional diameters for the deep lung may be greater than about 1-5 microns and may for example be about 1-10 microns. Similarly, particles with maximum cross-sectional diameters greater than about 5-10 microns such as about 5-30 microns may be used for delivery to the central airways. Coprecipitants which typically form bioactive molecule coated crystalline particles of this shape include histidine, and D,L-valine. For dry powder pulmonary formulations, particles made with coprecipitants that produce high aspect ratio flakes are therefore also preferred.

In particular, inhalable or pulmonary formulations may preferably be selected to have crystalline cores comprised of amino-acids such as valine, histidine, isoleucine, glycine or glutamine and which, for example, include: a crystalline core of valine and a coating of a therapeutic protein such as insulin; a crystalline core of histidine and a coating of an enzyme; a crystalline core of valine and a coating of an enzyme inhibitor such as $\alpha$ -antitrypsin; a crystalline core of valine and a coating of DNA; a crystalline core of valine and a vaccine coating; a crystalline core of glutamine and a vaccine coating; a crystalline core of glutamine and a coating of albumin. It is preferred when forming the particles for the formulation that co-precipitants are used which give discrete particles which do not aggregate on exposure to high humidity. In addition it is preferable that the coprecipitant does not leave an unpleasant taste in the patients mouth following administration. Glutamine is therefore highly preferred since it can be exposed to high humidity and has a bland taste.

According to a fourth aspect of the present invention there is provided a parenteral formulation comprising microcrystals or suspensions of microcrystals formed according to the first aspect.

Such formulations may be delivered by a variety of methods including intravenous, subcutaneous or intramuscular injection or else may be used in sustained or controlled release formulations. The microcrystals may be advantageously produced in a cost effective process to provide sterile parenteral formulations that exhibit extended shelf-life at ambient temperatures. Formulations in the form of powders or suspensions may be preferably reconstituted in aqueous solution in less than 60 seconds to provide low turbidity solutions suitable for injection. Reconstitution of suspensions may be preferred where the bioactive molecule is particularly toxic or potent and therefore difficult to manufacture or handle as a dry powder. Alternatively concentrated suspensions of microcrystals in a solvent such as, for example, ethanol or hydrofluorcarbons may be used for direct parenteral administration without reconstitution. This may provide advantages for bioactive molecules that require to be delivered at very high dosage forms to provide therapeutic effectiveness. Such bioactive molecules may include therapeutic antibodies and derivatives thereof. These may undergo aggregation on reconstitution or else may form highly viscous solutions that are difficult to administer. Concentrated suspensions of particles containing a high dosage of bioactive molecule may therefore be used to provide an alternative more convenient and therapeutically effective way of delivering such molecules. The suspension may be in solvent or else be prepared in a saturated aqueous solution of the coprecipitant used to prepare the microcrystals. Glutamine is a preferred coprecipitant for this application because of its low saturated concentration. Bioactive molecule coated particles are particularly suited to this application because on dilution they reconstitute very rapidly and show minimal aggregation of the bioactive molecule. Administration of aggregates is undesirable because it may lead to initiation of an adverse immune response.

Bioactive molecules suitable for administration by parenteral delivery include those described in the third aspect of this invention. In addition parenteral administration can be used to deliver larger biomolecules such as vaccines or antibodies not suited to administration into the subject's blood-stream via the lung because of poor systemic bioavailability. Preferred crystalline core materials include excipients commonly used in parenteral formulations such as mannitol and sucrose. Also preferred are natural amino-acids such as L-glutamine that can be used to form particles that reconstitute rapidly, are stable even at high temperature and are easy to process and handle. L-glutamine may also be preferred because it has been administered to patients at high dosages with no adverse side-effects.

According to a fifth aspect of the present invention there is provided a sustained or controlled release pharmaceutical formulation (or a depot) comprising microcrystals or suspensions of microcrystals according to the first aspect.

For certain applications it is preferable to produce parenteral or pulmonary formulations or other formulations that on administration provide sustained or extended therapeutic effects. This may, for example, be used to limit the maximum concentration of bioactive molecule that is attained in the subject's bloodstream or else be used to extend the period required between repeat administrations. Alternatively, it may be necessary to change the surface characteristic or solubility of the microcrystals to improve bioavailability or increase or decrease immunogenicity. The bioactive molecule coated microcrystals can be conveniently used to produce sustained or controlled release formulations. This can be achieved by coating the microcrystals or incorporating them in another matrix material such as a gel or polymer or by immobilising them within a delivery device.

For example, each of the microcrystals may be evenly coated with a material which alters the release or delivery of the components of the microcrystals using techniques known in the art.

Materials which may be used to coat the microcrystals may, for example, be: poorly water-soluble biodegradable polymers such as, for example, polylactide or polyglycolide and copolymers thereof; polyamino-acids; hydrogels; and other materials known in the art that change their solubility or degree of cross-linking in response to exposure to physiological conditions. The coating may for example be applied by contacting a suspension of particles with a solution of the coating material and then drying the resulting particles. If required the process can be repeated to extend the release profile. The coated microcrystals may be found to provide a substantially constant rate of release of the bioactive molecule into solution. Alternatively, a plurality of the particles may be combined into, for example, a single tablet form by, for example, by a binding agent. The binding agent may dissolve in solution whereupon the particles may be continually released into solution as the binding agent holding the tablet together progressively dissolves.

Those skilled in the art will realise that using combinations of the above teaching it is possible to provide other pharmaceutical formulations such as for example nasal formulations, oral formulations and topical formulations. Nasal formulations and oral formulations may require coating of the particles with alternate materials that provide adhesion to for example mucosal membranes.

According to a sixth aspect of the present invention there is provided a drug delivery device for inhalation comprising microcrystals formed according to the first aspect.

The inhalation may be pulmonary or nasal.

The pulmonary drug delivery device may, for example, be a liquid nebulizer, aerosol-based metered dose inhaler or dry powder dispersion device.

According to a seventh aspect of the present invention there is provided a method of determining optimum conditions for making bioactive molecule coated microcrystals comprising the steps of:

preparing a first batch of bioactive molecule coated microcrystals according to a first set of variable parameters and quantifying at least one property of said bioactive molecule coated microcrystals;
preparing a second batch of bioactive molecule coated microcrystals by changing at least one of the variable parameters, and quantifying said at least one property in order to be able to ascertain if said at least one change has a beneficial or detrimental effect on said at least one property.

Optionally, the method may be repeated as many times as necessary in order to seek to obtain microcrystals which display optimal properties.

This method may be conveniently used to screen either batch or continuous processes to find optimal conditions for producing bioactive molecule coated microcrystals. A three-stream continuous flow precipitator is found to provide a particularly convenient and efficient method for implementing such a screening process. The aim of the screen is generally to provide particles with the most attractive physical properties for a particular application while at the same time retaining maximum bioactivity but other screens are also possible.

The screening process may essentially consist of

a) identifying what properties of the bioactive molecule coated microcrystals are most desirable for a particular application and ensuring these properties can be accurately quantified by some type of measurement;
b) identifying those parameters expected to most effect the properties that need to be optimised;
c) determining over what ranges the identified parameters will be varied and what constraints there are on practically achieving this;
d) identifying a matrix of experiments that will efficiently screen the effect of the chosen parameters on the desired properties;
e) carrying out the coprecipiations identified by the experimental design process and making measurements of the properties exhibited by the coated microcrystals produced;
f) analysing the results obtained and if necessary designing further experiments to find the optimal conditions;
g) optionally repeating a)-f) until a process for making microcrystals fits for the identified purpose.

Examples of physical properties that may be screened include mean size, mean shape, size distribution, crystallinity, dissolution time, mean aerodynamic diameter, total emitted dose, fine particle fraction and other measurable parameters that may be relevant to pharmaceutical formulations. Examples of properties relevant to retention of bioactivity include yield of bioactive molecule retained on crystals, loading or doseage of bioactive molecule, turbidity, degree of aggregation, *in vitro* bioactivity, absence of cleavage products, *in vitro* bioavailability, level of impurities, retention of structure and other parameters that may be relevant to therapeutic applications. Measurements relevant to desired physical properties or bioactivity may take place immediately following preparation of the bioactive coated microcrystals or else following a storage period in which the samples may be deliberately stressed by exposure to, for example, elevated temperatures or humidity.

Surprisingly it is found that the screening process can generally be carried out most efficiently with a three-line continuous flow precipitator.

The successful co-precipitation of bioactive molecule coated microcrystals in a continuous process using a particular coprecipitant/solvent combination has been found to depend on several process parameters, and in most cases it may be necessary to screen and optimise these factors. There may be at least three main parameters, which should preferentially be screened and optimised, these may be:

- Protein Loading (%w/w)
- Final Water Content (%v/v)
- Coprecipitant Concentration (mg/ml)

Protein loading and final water content may be found to influence the particle size and level of retained bioactivity. Initial coprecipitant concentration may be found to affect the supersaturation achievable and nucleation profiles, which in turn may be found to affect particle size and shape and particle aggregation. Hence, if bioactive molecule coated microcrystals are required for a particular application it may be found that screening of these three parameters can provide a rapid route to reaching an initial formulation.

Other parameters that may also be found to affect bioactivity and/or particle characteristics include:

- pH

- Temperature (Reagent Solutions; Mixing cell; Production Solution)

- Additives/Stabilisers

- Mixing efficiency

Optimisation using these parameters may for example be carried out during a second round screening process. This parameter list is not exhaustive, but serves to illustrate some additional parameters that can potentially be investigated.

Investigating the effect of these parameters can be approached in at least two ways. Firstly there is the simple method of screening each parameter individually, holding all other parameters constant. This iterative approach, although acceptable, is often inefficient in terms of resources and generally very time-consuming. Furthermore, long-term investigations may be susceptible to systematic and random error.

The second approach utilises a more efficient statistical approach, whereby the majority of the parameters may be firstly screened, followed by a careful optimisation of the most influential parameters. This method is commonly referred to as Design of Experiment (DoE) and has been found to offer significant advantages. In a typical study, five parameters may be identified. For example, if particles with a particular characteristic are required using a target bioactive molecule/coprecipitant/solvent combination then the first step may be to screen against three of the fundamental factors identified previously, such as for example protein loading, final water content and coprecipitant concentration. Additionally, prior knowledge might indicate that the bioactive molecule (e.g. a protein) may be likely to be sensitive to pH and temperature. In the initial screen therefore, five parameters may be investigated.

For each parameter a sensible experimental range may be identified. Often this relies on knowledge obtained from previous similar investigations, but it may require an estimation of a realistic, accessible level. Typically, for example, a reasonable protein loading range for testing a formulation may be about 0.25 - 10% w/w. Similarly, a typical final water content range may be about 0.5 - 10% vv, and a typical excipient concentration range may be about 75 - 90% of saturated concentration. For example, if DL-valine has a saturated concentration of around 66 mg/ml at a particular temperature then 75% equals 50 mg/ml; 90% equals 60 mg/ml. In the same way, a reasonable pH and temperature range may be chosen. It may be preferable to try and predict ranges that will given an appreciable change in response. Often it may be necessary to initially start with quite a wide range, such as one that spans an order of magnitude. Again, previous knowledge will help in this selection process. Furthermore, the parameter ranges selected need to give measurable responses in the subsequent sample analyses. Therefore, if the analytical techniques are very sensitive, capable of reporting very small changes between samples, then it may be acceptable to select narrow parameter ranges. On the other hand, if the analytical techniques are somewhat insensitive or are compromised by high levels of noise, it may be wiser to select a wide parameter range.

After selection of parameters and suitable ranges, it may be necessary to input the chosen factors into the DoE software. Recently, there has been a significant increase in the amount of DoE software available, and many are available on-line for trial purposes. The inventors have reviewed a handful of examples, and have concluded that for simple parameter investigation, all are acceptable. All software packages have similar DoE input criteria, diversifying only in post-experimental statistical analysis.

In the scope of the present application, the DoE package MODDE 6, supplied by Umetrics was used. This package is attractive in that basic usage is very simple. The experimenter may be prompted to input the experimental parameters chosen (termed in the software as factors), and the analytical measurements that will be measured (termed in the software as responses). Thereafter, the experimenter may be asked if the study in question is a screening study or an optimisation study. Depending on the choice made, a selection of possible statistical studies may be suggested, and the scientist may be prompted to select the most suitable one. Finally, a matrix of experiments and analyses may be produced, providing a systematic scheme of work.

The experimenter may now be ready to perform the experiments, preferably in a randomised manner, as this may minimise the effects of random error in the eventual statistical analysis.

According an eighth aspect of the present invention there is provided a tuneable method of forming bioactive coated microcrystals for delivering to a particular area of a lung comprising:

(a) providing a first aqueous solution comprising coprecipitant molecules;
(b) providing a second aqueous solution comprising bioactive molecules;
(c) providing a third solution comprising water miscible solvent;
(d) mixing said first aqueous solution, said second aqueous solution and said third solution substantially simultaneously; or mixing either the first and second aqueous solutions with the third solution and thereafter mixing with the remaining of either the first and second aqueous solutions; such that coprecipitation of the coprecipitant and the bioactive molecules is initiated leading to formation of said microcrystals;
(e) collecting a suspension of said microcrystals; and
(f) detecting the average size of the microcrystals and determining whether or not such microcrystals would be suitable for delivering to a particular area of a lung, and in the event that the average particle size of the microcrystals is not suitable, altering variable parameters in the tuneable method.

Parameters that may affect the size of particles formed include concentration of coprecipitant, pH, solvent, water content, bioactive molecule loading, temperature, additive concentration and mixing efficiency. If sequential addition of the first and second aqueous solutions to the solvent is used the time for the solution to pass from the first mixer to the second mixer may also be varied. In some sytems one or more of these values may necessarily be fixed but DoE may be used to carry out an efficient screen of the remaining variable parameters. It can be used to identify those parameters that most affect the size and any interactions between them. This information can then be used to further refine the parameters via response surface modelling to generate a particle size within a target range while at the same time also optimising bioactivity and the efficiency of binding.

According to a ninth aspect of the present invention there is provided a method of receiving a request to form bioactive molecule coated microcrystals according to any previous aspect comprising:

(a) a customer defining a requirement for bioactive molecule coated microcrystals including that of bioactivity, dosage, loading, size, shape and coprecipitant;
(b) tuning the coprecipitation conditions in order to obtain microcrystals as defined in part (a);
(c) refining the coprecipitation conditions to obtain microcrystals with appropriate requirements; and
(d) identifying appropriate conditions to form the required microcrystals.

Typically, the coprecipitation conditions which may be altered may be selected from any of solvent, pH, temperature, rate of mixing and concentration.

During the refining process, a stability study may also be performed on the bioactivity and integrity of the microcrystals.

In the event that the obtained microcrystals are not suitable, steps (b) and (c) may be repeated.

According to a tenth aspect of the present invention there is provided a method of providing services relating to a method of forming bioactive molecule coated microcrystals according to any previous aspect, said method comprising:

receiving a request from a customer to form bioactive molecule coated microcrystals with particular requirements including that of size and bioactivity;
determining a method of forming said bioactive molecule coated microcrystals; and
receiving payment for providing services of said method of forming bioactive molecule coated microcrystals to the customer.

## Brief Description of the Drawings

Embodiments of the present invention will now be described, by way of example only, with reference to the accompanying drawings in which:

Figure 1 is a graph representing the results of a bicinchoninic acid (BCA) assay for fresh trypsin as received from supplier and trypsin PCMCs produced by a two-line continuous flow co-precipitator and a three-line continuous flow co-precipitator;
Figure 2 is a graph showing the results of a N-p-tosyl-L-arginine methyl ester (TAME) assay for fresh trypsin and trypsin PCMCs produced by a two-line continuous co-precipitator and a three-line continuous co-precipitator;
Figure 3 is a graph of BCA/TAME corrected activity data for fresh trypsin, and PCMCs produced by a two-line continuous flow co-precipitator and a three-line continuous flow co-precipitator;
Figure 4 shows particles sizes obtained from dynamic light scattering (DLS) of trypsin coated and uncoated valine microcrystals produced by a two-line continuous flow co-precipitator and a three-line continuous co-precipitator;
Figure 5 is a graph showing the results of the bicinchoninic acid (BCA) protein loading analysis for a range of PCMCs and fresh trypsin;
Figure 6 is a graph showing TAME results for the retained enzymatic activity of trypsin/DL-valine PCMCs relative to fresh trypsin;
Figure 7 is a graph showing BCA corrected retained activity for trypsin/D,L-valine microcrystals;
Figure 8 shows particle sizes obtained from dynamic light scattering (DLS) of trypsin coated and uncoated valine microcrystals obtained using different coprecipitant concentrations
Figure 9 is a representation showing different rates of sedimentation of PCMCs formed in a two-line continuous flow co-precipitator and a three-line continuous flow co-precipitator;
Figure 10 shows particle size ranges for PCMCs formed in a two-line continuous flow co-precipitator;
Figure 11 shows particle size ranges for PCMCs formed in a three-line continuous flow co-precipitator;
Figure 12 is an SEM image of bovine IgG coated microcrystals formed according to the present invention; and
Figure 13 is an image showing different parts of a lung.

## Experimental Results

## Example 1

Co-procipitation of Protein Coated Micro Crystals (PCMCs) using a Three-Line Continuous Flow Co-precipitator (CFCP).

Overview

In PCT/GB2004/000044, continuous flow co-precipitation of PCMCs using a two-line system was used, whereby an aqueous solution of protein and excipient were continuous blended with a miscible solvent. In the present application, PCMCs are produced by a three-line system, whereby the three components of PCMCs, namely the protein, the coprecipitant and the miscible solvent are each delivered independently. Pump A delivered an aqueous buffered solution of protein, pump B delivered an aqueous solution of coprecipitant and pump C delivered the miscible solvent.

The experimental procedure and the results obtained are derailed below. The experiment is labelled JV714.

The objective of this experiment was to compare theoretically identical PCMCs made firstly by a two-line system and secondly by a three line system.

For the two-line system, the CFCP was used as previously reported in PCT/GB2004/000044. All pumps were calibrated before use. For the three-line system, the CFCP was modified to include another pump, which was plumbed into the mixing cell through an additional entry post. Flow rates were adjusted accordingly, as reported below, but otherwise all other parameters remained constant.

From previous studies it was decided to use trypsin-DL-valine PCMCs as the model system. A theoretical 2.5 %w/w loading of trypsin on DL-Valine was prepared. The water content of the PCMC suspension was fixed at 4.0 %v/v. The total flow rate was fixed at 100 ml min$^{-1}$. Trypsin (Lot. No> 81K7653) and DL-Valine (Lot. No. 55H1252) was obtained from Sigma Aldrich Co. UK. Propan-2-ol (Lot. No. K32707146347) and calcium chloride (Lot. No. 9930948 389) was obtained from BDH, Poole, UK. Calcium chloride has been discovered to improve retention of trypsin on the surface of the PCMC crystal and was therefore included in this experiment. It may be introduced via the aqueous or organic solvent but in this experiment was included in the aqueous coprecipitant mixture.

The following tables details the flow rates, protein and/or coprecipitant concentration delivered by each pump used.

Table 1

| Number of Lines | Pump A Solute & Flow Rate | Pump B Solute & Flow Rate | Pump C & Solute Flow Rate | Total Flow Rate |
|---|---|---|---|---|
| Two | Trypsin (1.538 mg/ml) + DL-Valine (60 mg/ml) + CaCl$_2$ (2.19mg/ml) @ 4 ml min$^{-1}$ | Propan-2-ol saturated with DL-Valine @ 96 ml min$^{-1}$ | - | 100 ml min$^{-1}$ |
| Three | Trypsin (12.304mg/ml) @ 0.5 ml min$^{-1}$ | DL-Valine (68.571 ml min$^{-1}$) + CaCl$_2$ (2.5mg/ml) @ 3.5 ml min$^{-1}$ | Propan-2-ol saturated with DL-Valine @ 96 ml min$^{-1}$ | 100 ml min$^{-1}$ |

It was necessary to ensure that per unit time, exactly the same amount PCMC component entered the mixing cell.

For both the two-line and three-line experiments, a single batch of miscible solvent was prepared as follows. Excess DL-valine was added to approximately 2 1 of propan-2-ol, and this solution was agitated at room temperature for approximately two hours at room temperature. Thereafter the solution was immediately filtered through a 0.45 $\mu$m membrane filter. *This solution will be referred to as Solution 4.*

For the two-line experiment, the trypsin-DL-valine solution was prepared as follows. A stock solution of 2.19 mg/ml CaCl$_2$ in deionised water was prepared. Using this stock solution, a solution containing 60 mg/ml DL-valine was prepared. Finally, trypsin was added to give a concentration of 1.538 mg/ml. *This solution will be referred to as Solution 1.*

For the three line experiment, two solutions were prepared. A stock solution of 2.5 mg/ml CaCl$_2$ in deionised water was prepared. Using this stock solution, a solution containing 68.571 mg/ml DL-valine was prepared. *This solution will be referred to as Solution 2.*

Also a solution of trypsin in deionised water, concentration 12.304 mg/ml, was prepared. *This solution will be referred to as Solution 3.*

The two-line PCMCs were prepared as follows. Solution 1 was blended with Solution 4 at flow rates of 4 and 96 ml min$^{-1}$ respectively. 25 ml of PCMC was collected. Approximately 5-10 minutes after co-precipitation, PCMCs were immediately filtered over a 0.45 $\mu$m membrane filter, and left to dry in an incubator @ 30°C for approximately 1 hour.

The three-line PCMCs were prepared as follows. Solutions 2, 3 were blended with Solution 4 at flow rates of 0.5, 3.5 and 96 ml min$^{-1}$, respectively. 25 ml of PCMC was collected. Approximately 5-10 minutes after coprecipitation, PCMCs were immediately filtered over a 0.45 $\mu$m membrane filter, and left to dry in an incubator @ 30°C for approximately 1 hour.

Analysis

The samples prepared were analysed using three analytical techniques. Firstly, the protein loading of trypsin on DL-valine was measured using the bicinchoninic acid (BCA) colourimetric assay. Secondly, the activity of the trypsin-DL -valine PCMCs was ascertained using a well-known trypsin assay - the hydrolysis of N-p-tosyl-L-arginine methyl ester (TAME) hydrochloride assay. Thirdly, the size and span of the PCMC particle population was measured using a dynamic light scattering (DLS) analyser, namely a Malvern Mastersizer.

BCA Assay

Approximately 5 mg of dried material was accurately weighed into a 25 ml vial. Theoretically the protein loading should have been 2.5 %w/w, therefore the weight weighed was multiplied by 2.5 %w/w. The resultant value represented the theoretical amount of trypsin in the PCMC sample. This value was divided by 0.015 mg/ml (assaying concentration) to give the required dilution. PCMCs were dissolved in assaying buffer (0.046M Tris; 0.0115M $CaCl_2$ pH 8.1@25°C). Each solution was then analysed as follows. Add 0.5 ml of protein standard into 1.5ml Eppendorf. Add 0.5 ml of BCA working buffer to each Eppendorf. (A precipitate may form). Centrifuge for 2 minutes @ 12,000 rpm. Place in heating block @ 60°C for exactly 15 minutes. Centrifuge for 2 minutes @ 12,000 rpm.
Pipette 0.75 ml into a micro-cuvette and read at 562 nm immediately.

TAME Assay

Approximately 5 mg of dried material was accurately weighed into a 25 ml vial. Theoretically the protein loading should have been 2.5 %w/w, therefore the weight weighed was multiplied by 2.5 %w/w. The resultant value represented the theoretical amount of trypsin in the PCMC sample. This values was divided by 0.015 mg/ml (assaying concentration) to give the required dilution. PCMCs were dissolved in water. Each solution was analysed as follows. Into a clean UV quartz cuvette was added 1.3 ml assaying buffer and 150 μl 0.01M TAME solution. Incubate for 5-10 minutes at 25°C. Add 50 ul of PCMC diluent, agitate and assay at 247 nm for 10 minutes.

Dynamic Light Scattering Analysis(DLS)

Samples were analysed as PCMC suspension and as dry powders that had been reconstituted in DL-valine saturated anti-solvent. Instrument was first blanked with anti-solvent. Thereafter approximately 500 μl of PCMC suspension was added to give a laser obscuration of approximately 12%. Five replicate measurements of each sample were made.

BCA Assay

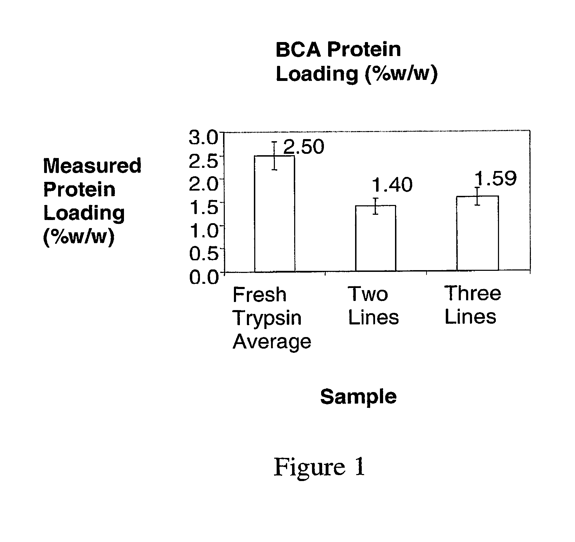

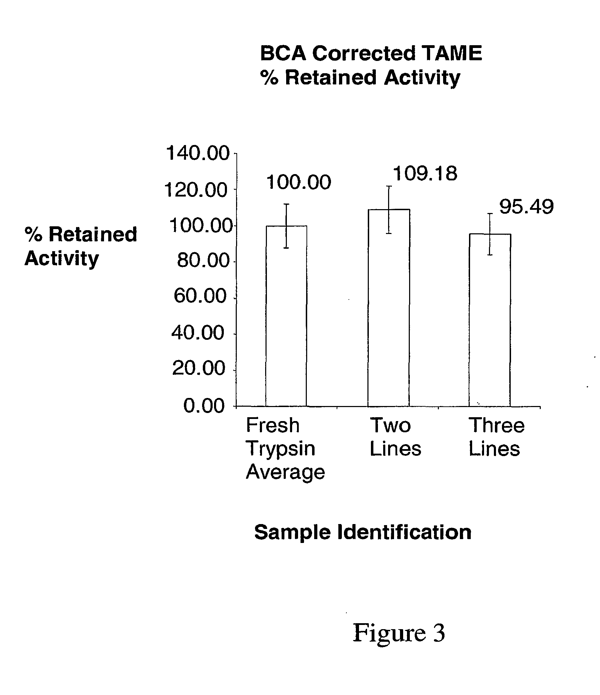

Figure 1 is a graph showing the results of the BCA assay for fresh trypsin as received from supplier; trypsin PCMCs produced by two line CFCP and three line CFCP respectively.
The BCA results of Figure 1 show that, within experimental error, the two line and three line methods do not differ significantly.

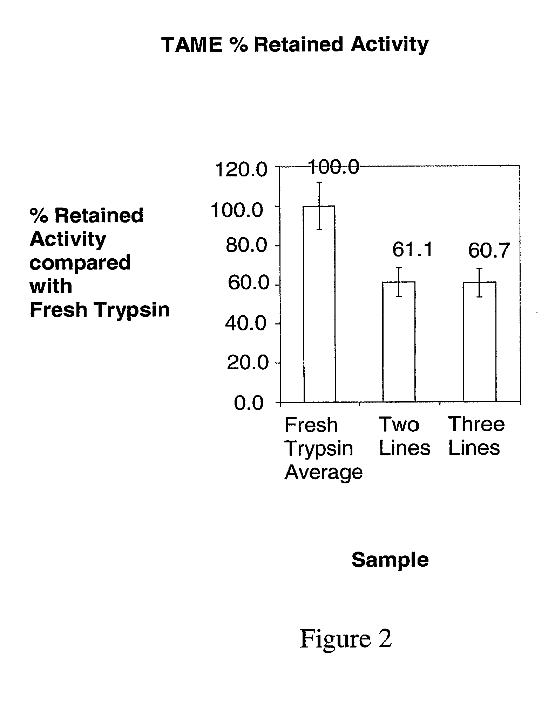

Figure 2 is a graph showing the results of the TAME assay for fresh trypsin as received from supplier; trypsin PCMCs produced by two line CFCP and three line CFCP respectively.
Like the BCA assay previously, the TAME results also show that, within experimental error, the two-line and three-line systems do not different significantly. Figure 3 shows the effect of correcting the activity of the PCMCs (as determined by TAME assay) by the protein loading of the PCMCs (as determined by the BCA assay). Dynamic Light Scattering (DLS)

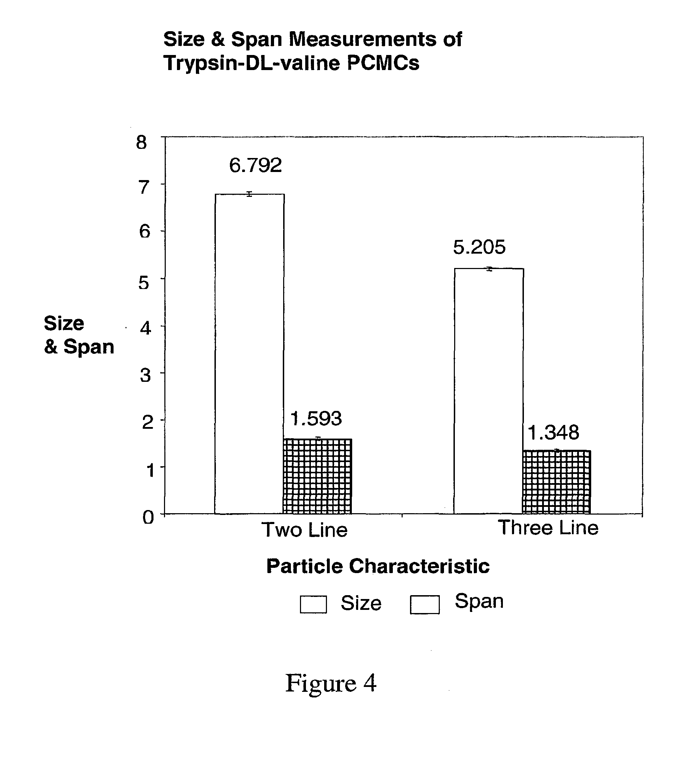

Figure 4 shows Dynamic Light Scattering (DLS) results which show that microcrystals produced by the three-line system are measurably smaller in size and span.

Conclusions

Trypsin-DL-valine PCMCs were produced by two-line and three-line co-precipitation methods. All other parameters were held constant. The protein loading, activity, size and span of the PCMCs were measured. From the bioactivity results obtained, the two-line and three-line systems do not differ significantly but the particles obtained were found to be smaller. This may be advantageous for pharmaceutical formulations.

Example 2

In this example, three lines were used to coprecipitate trypsin/DL-valine PCMCs using coprecipitant solutions held at different temperatures. The theoretical protein loading was 2.5% w/w; the water content was 4.0% v/v; the total flow rate was 100 ml/min. In one experiment [JV790] the aqueous DL-valine solution was heated and the concentration was 90 mg/ml and in another [JV675] the DL-valine solution was held at room temperature and the concentration was 68.571 mg/ml. Calcium chloride was not included in these experiments. In order to dissolve 90 mg/ml DL-valine in deionised water, it is necessary to heat and maintain the solution temperature at ~90°C. Solutions were heated using a Techne Dri-Block DB-3 Heating block. This unit is thermostatically controlled, and maintains constant temperature.

The theoretical protein loading was 2.5% w/w and the excipient concentration was 90 mg/ml. In a comparative experiment where all lines were held at room temperature the theoretical protein loading was 2.5 % w/w, but the excipient concentration was 60 mg/ml. As a consequence of this it was necessary to adjust the solution concentrations, as shown in Table 2 below:

Table 2

| Solution | JV675 | JV790 |
|---|---|---|
| Solution 2 (DL-Valine in deionised water) | 68.571 mg/ml* @ 25°C | 90 mg/ml* @ 90°C |
| Solution 3 (Trypsin in deionised water) | 12.304 mg/ml @ 25°C | 16.152 mg/ml @ 25°C |
| Solution 4 (DL-valine saturated propan-2-ol) | Saturated DL-Valine in IPA | Saturated DL-Valine in IPA |

*Table 2 details the concentrations of trypsin and excipient required to produce trypsin / DL-Valine PCMCs, using the three line system.*

*\*These numbers refer to the coprecipitant concentration in the unmixed solutions. When the three solutions mix the effective aqueous concentration or [coprecipitant]$_{aq}$ will depend on relative flow rates. The constituent flows of solutions 2 and 3 were 3.5 and 0.5 ml min$^{-1}$ respectively. The total aqueous flows of solution 2 and 3 were 4.0 ml min$^{-1}$. Consequently at the point of the mixing, the following calculation gives [coprecipitant]$_{aq}$;*

$$[coprecipitant]_{aq} = \frac{Pre\text{-}mixing\ Concentration\ (mg/ml)\ x\ Premixing\ Flow\ Rate\ (ml/min)}{4.0\ (ml/min)}.$$

*Thus, in JV675 the effective aqueous concentration of D,L-valine, [coprecipitant]$_{aq}$, is 60mg/ml and in JV790 it is 78. 75 mg/ml.*

In summary, therefore, this additional experiment investigates the effect of increasing the effective DL-Valine concentration from 60 mg/ml to 78.75 mg/ml. All other parameters were held constant, and all samples were analysed for protein loading (BCA), enzymatic activity (TAME) and particle size (DLS) as described previously.

Samples were prepared in a similar fashion to previous three line samples, however because the excipient solution was thermostatically controlled at 90°C it was essential to try and maintain this temperature till the point of mixing. Pre-flushing the cell with deionised water at 90°C hopefully ensured that the temperature drop due to conductance in the metal piston was minimized. No pump blockage issues were experienced during this experiment.

Samples were co-precipitated, and approximately 10-15 ml of the suspension was filtered on the Millipore Durapore filters. Immediately after filtration, the sample was dried in an incubator for no less than 16 hours. The samples prepared and analysed are shown in Table 3.

Table 3

| Sample ID | Protein (%w/w) | Effective D,L-valine Concentration (mg/ml) |
|---|---|---|
| JV675/3 | 0 | 60 |
| JV790/2 | 0 | 78.75 |
| JV675/4 | 2.5 | 60 |
| JV790/4 | 2.5 | 78.75 |

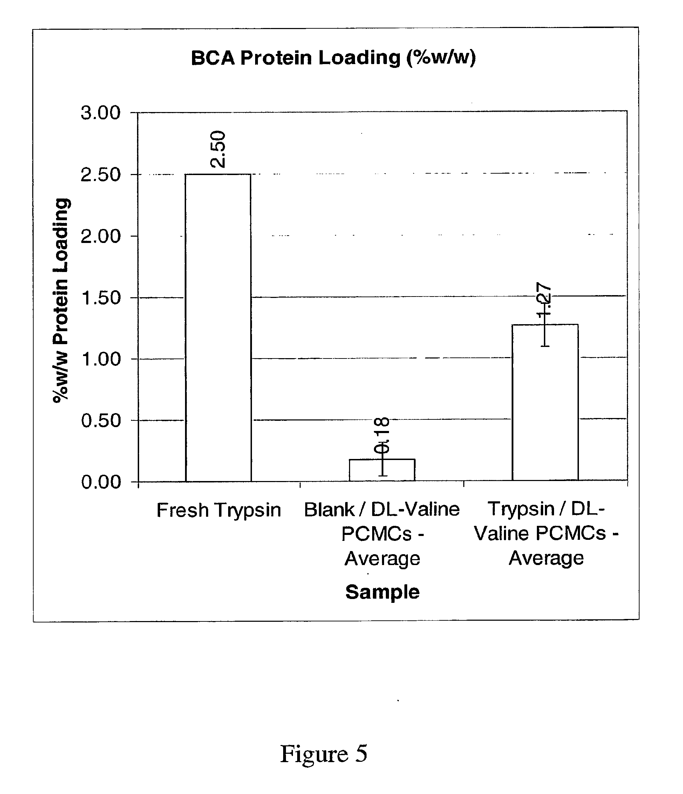

Figure 5 is a graph showing the results of the BCA protein loading analysis for JV790/2, JV790/4, including fresh trypsin as received from supplier. There is clearly some loss of trypsin during the coprecipitation process as expected when calcium chloride is not present

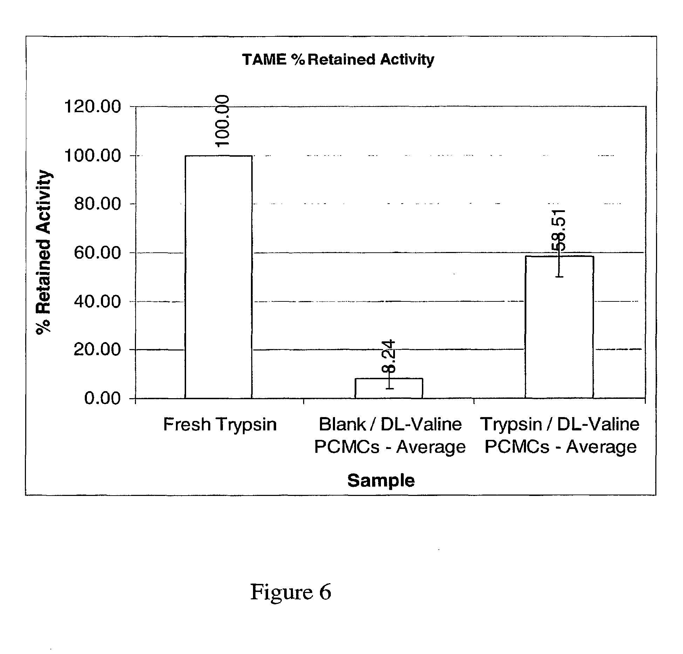

Figure 6 is a graph showing the enzymatic activity of JV790/2 and JV790/4, including fresh trypsin as received from the supplier. The loss of retained bioactivity for JV790/4 is mainly due to loss of protein.

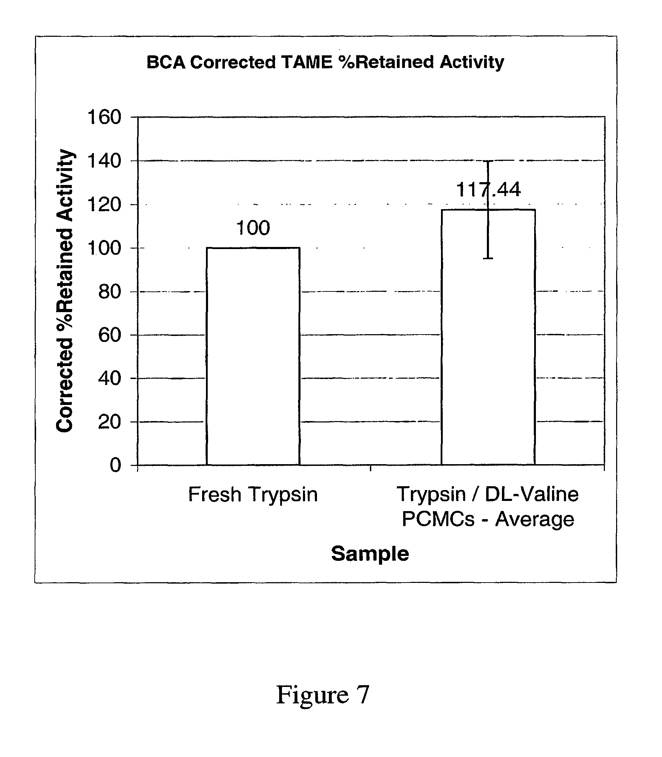

Figure 7 is a graph of bioactivity obtained in JV790 that has been corrected for actual protein loading measured using the BCA assay. It can be seen that the trypsin present has high retained activity.

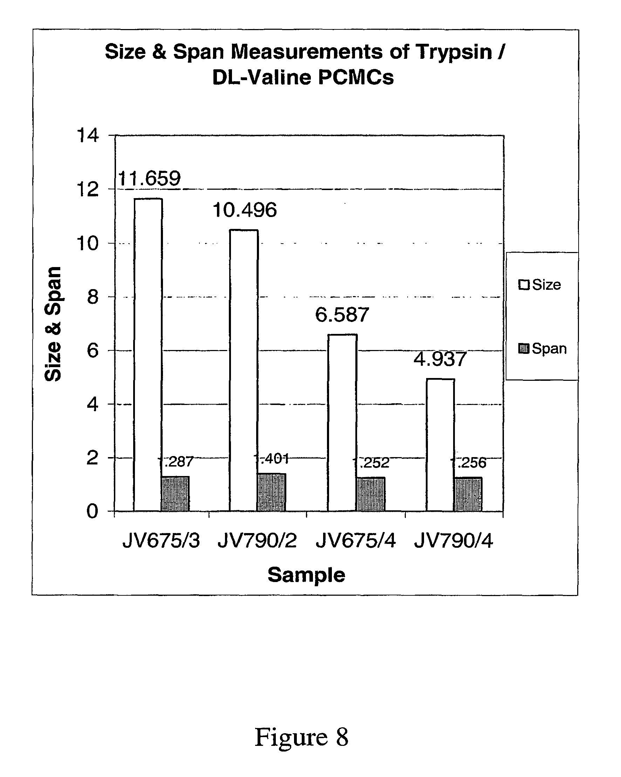

Figure 8 is a graph showing the results of the Malvern Mastersizer analysis. For both the JV675 and JV790 experiments it can be seen that samples prepared with trypsin are much smaller than samples without trypsin. Comparing the JV675 and JV790 results the increased coprecipitant concentration in JV790 has led to a decrease in particle size. This is most clearly seen for the samples prepared with the bioactive molecule, trypsin, present. Samples prepared at the higher coprecipitant (JV790) are 25% smaller than those prepared at the lower concentration (JV675). The results demonstrate the advantage of separating the aqueous coprecipitant solution from the bioactive molecule solution. In this case the D,L-valine solution could be heated to 90 °C without running the risk of denaturing the trypsin.

Conclusions

In general trypsin/DL-valine PCMCs produced using an excipient concentration of 90 mg/ml at a temperature of 90°C did not alter the protein loading and enzymatic activity characteristics relative to those obtained using comparable room-temperature conditions. This is shown in Figure 7. The experimental hypothesis proposed that high coprecipitant concentrations would induce higher supersaturations and hence smaller PCMCs. This was found to be the case when protein was present and a 25% size decrease in the particle size was observed using the heated, higher concentration coprecipitant solution.

## **Example 3**

Comparison of a 2 line or 3 Line CFCP system for coprecipitation of Trypsin / $K_2SO_4$ into isopropanol, [Expt JV818].

This example was designed to determine if the previously described advantages of using a 3 line mixing process could also be obtained with coprecipitants known to coprecipitate very rapidly.

$K_2SO_4$ is an inorganic salt, which rapidly precipitates from a concentrated aqueous solution upon addition to a suitable anti-solvent such as propan-2-ol. In the literature it is well known that inorganic salts precipitate rapidly, and in many cases precipitation is so quick, that even measuring the process is difficult. Previously we have demonstrated that bioactive molecule coated microcrystals may be made with potassium sulfate using either a batch system or a two line continuous process. Further it has been consistently found (with $K_2SO_4$ and many other materials) that the coprecipitation process leads to the formation of crystals smaller than those obtained on precipitation in the absence of protein. Particle size therefore provides a convenient diagnostic tool for determining if the bioactive molecule has affected the formation of crystals during coprecipitation.

In Example 1 it was shown that for DL-valine coprecipitated with trypsin by either a 2 line or 3 line process there is a significant reduction in particle size relative to precipitation of pure DL-valine. In this example a 3 line mixing system was applied to coprecipitation of the bioactive molecule trypsin with potassium sulfate ($K_2SO_4$) and changes in crystal size used to determine if the product differed from that obtained with a two-line system.

## **Experimental procedure.**

The following series of samples were prepared, as shown in Table 4.

Table 4

| Sample No | Protein | Lines | Retention |
|---|---|---|---|
| 1 | No Protein | Two | Suspension |
| 2 | Protein | Two | Suspension |
| 3 | No Protein | Three | Suspension |
| 4 | Protein | Three | Suspension |

For this experiment it was necessary to calculate necessary flow rates and the required reagents, as shown in Table 5.

Table 5

| Sample No. | Number of Lines | Pump A Solute & Flow Rate | Pump B Solute & Flow Rate | Pump C Solute & Flow Rate | Total Flow Rate |
|---|---|---|---|---|---|
| 1 | 2 | 100 mg/ml $K_2SO_4$ dissolved in deionised water @ 4 ml min$^{-1}$ | Propan-2-ol saturated with $K_2SO_4$ @ 96 ml min$^{-1}$ | - | 100 ml min$^{-1}$ |
| 2 | 2 | 2.56 mg/ml trypsin & 100 mg/ml $K_2O_4$ dissolved in deionised water @ 4 ml min$^{-1}$ | Propan-2-ol saturated with $K_2SO_4$ @ 96 ml min$^{-1}$ | - | 100 ml min$^{-1}$ |
| 3 | 3 | Deionised water @ 0.36 ml min$^{-1}$ | 110 mg/ml $K_2S0_4$ dissolved in deionised water @ 3.64 ml min$^{-1}$ | Propan-2-ol saturated with DL-$K_2SO_4$ @ 96 ml min$^{-1}$ | 100 ml min$^{-1}$ |
| 4 | 3 | 28.204 mg/ml trypsin dissolved in deionised water | 110 mg/ml $K_2S0_4$ dissolved in deionised water @ 3.64 ml min$^{-1}$ | Propan-2-ol saturated with DL-$K_2SO_4$ @ 96 ml min$^{-1}$ | 100 ml min$^{-1}$ |

A theoretical protein loading of 2.5 %w/w was selected. The water content of the PCMC suspension was fixed at 4.0 %v/v. The total flow rate was fixed at 100 ml min$^{-1}$. Trypsin (Lot. No. 121K7692) and $K_2SO_4$ (Lot. No. 121K0043) was obtained from Sigma Aldrich Co. UK. Propan-2-ol (Lot. No. K32883646) was obtained from BDH, Poole, UK.

As in previous experiments, it was necessary to prepare more concentrated solutions of trypsin and $K_2SO_4$ for the 3-line system so as to ensure the same amount of material was entering the flow cell per unit time. Flow rates were also altered to ensure the water content was fixed at 4 %v/v. The stirrer speed was fixed at 1500 rpm.

The CFCP system was calibrated before coprecipitations were conducted. Each pump was calibrated independently, and it was found that the pumps were satisfactorily accurate.

Samples 1-4 were coprecipitated and immediately transferred to the fridge for storage.

Results

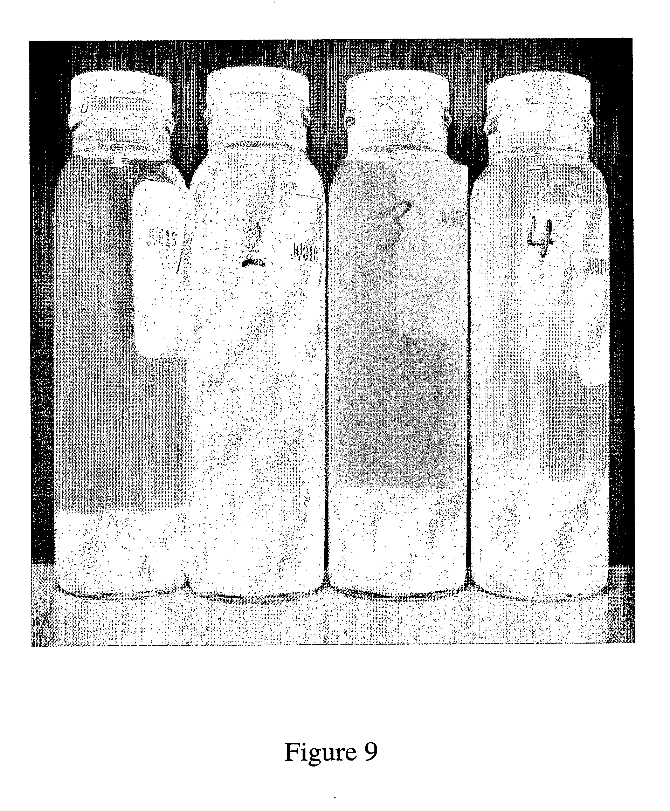

Almost immediately after coprecipitation it was clear that there was an observable difference between the 2- and 3-line systems. Comparing samples 2 and 4, it was observed that in sample 2 the particles were significantly smaller than sample 4 particles, as the rates of sedimentation differed dramatically. In fact sample 4 was essentially similar to samples 1 & 3 that do not contain trypsin. Figure 9 shows the extent of sedimentation approximately 10 minutes after coprecipitation.

Clearly, sample 3 differs significantly from the others, which suggests that only in sample 2 has the trypsin interacted with the $K_2SO_4$ to produce PCMCs.

Approximately 4 hours after coprecipitation, all suspension samples were analysed using a Malvern Mastersizer, that uses Laser diffraction technology to ascertain size and span of the particles produced. The results are shown on Table 6.

Table 6

| Sample | Size (d 0.5; $\mu$m) | Span |
|---|---|---|
| 1 | 11.0885 | 0.9615 |
| 2 | 0.240 | 39.162 |
| 3 | 11.623 | 1.030 |
| 4 | 10.002 | 1.133 |

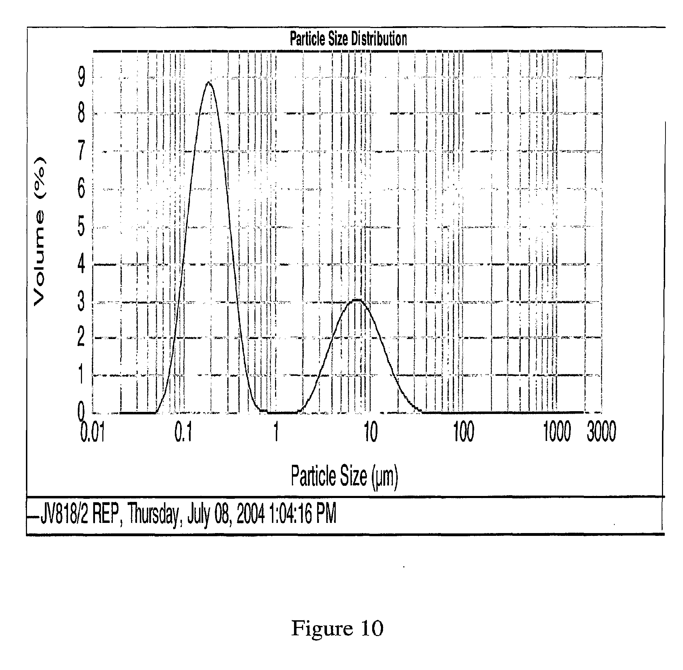

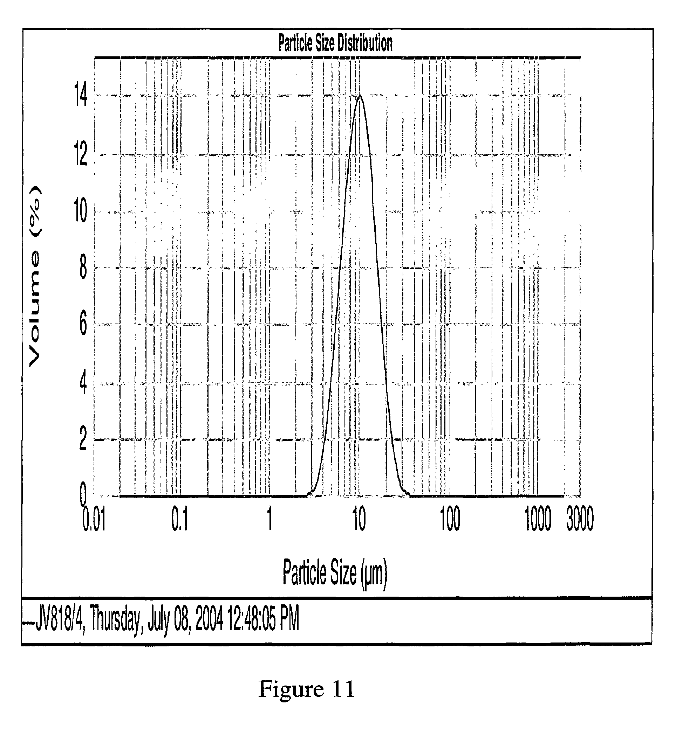

Essentially samples 1, 3 and 4 are the same within experimental error. Sample 2 however, is largely made up of particles that are significantly smaller, consistent with the observation that the sample takes a longer time to settle. Figures 10 and 11 show the Mastersizer results for samples 2 and 4, respectively.

Figure 10 demonstrates that bioactive molecule coated microcrystals with sub-micron dimensions are accessible provided the appropriate mixing regime is applied. Figure 11 shows that although the mixing was too slow in the 3 line system to provide for interaction of the protein during the initial precipitation the particles are nevertheless all of a similar size. It is probable that with a pI of 9 the trypsin electrostatically binds to precipitated $K_2SO_4$ crystals which have a negative Zeta potential. This provides a route to larger coated particles with a narrow size distribution that may find useful applications in production of slow-release formulations.

Conclusions

These results show clearly that for trypsin coprecipitated with $K_2SO_4$ the particles obtained in the three line system are identical in size to particles obtained by precipitation of pure $K_2SO_4$. On the other hand in the 2 line system much smaller particles are obtained in the presence of protein. The differences between the results obtained here and those obtained with DL-valine are striking and can be correlated with the relative rates of precipitation. With valine near complete mixing can be attained in the 3 line system prior to the onset of precipitation. This allows the bioactive molecule to intervene in the particle nucleation process. In this case particles prepared in the 3 line system were measurably smaller than those obtained in the 2 line system and significantly smaller than those obtained without protein.

With $K_2SO_4$ precipitation is very fast and so if the two aqueous streams are introduced separately then particle formation occurs before complete mixing occurs and addition of the protein has no effect. This demonstrate the important active role the bioactive molecule plays in controlling the size of particles. It also demonstrates that to obtain the advantages of a 3 line system in terms of being able to keep the two aqueous components separated it is necessary to choose coprecipitants such as amino-acids or sugars that coprecipitate on a slower time-scale than that required to achieve good mixing.

**Example 4**

Preparation of antibody coated microcrystals by a 2 line and 3 line process

Bovine IgG-coated valine microcrystals were prepared by a 2 line and 3 line process using the equipment described in Example 1 and conditions described in Table 7. The supplier of Bovine IgG (Lot 052742366) at 10mg/ml in 0.01M sodium phosphate, 0.15M NaCl (PBS), pH 7.4 (Preservative 0.1% NaAzide) was Lampire Biologicals, PO Box 270, Pipersville, PA 18947. For both the 2 line and 3 line experiment the conditions are designed to produce the same theoretical protein loading in the formulation of 7.5 wt%, a final water content in the suspension of 4 %v/v and the same supersaturation of valine during precipitation. The key difference between the two experiments is that in the 3 line experiment the protein is introduced into the coprecipitation in PBS buffer at pH 7.4 while in the two line experiment it is mixed with the valine coprecipitant and at a pH of around 6.2.

Table 7

| Number of Lines | Pump A Solute & Flow Rate | Pump B Solute & Flow Rate | Pump C Solute & Flow Rate | Total Flow Rate |
|---|---|---|---|---|
| Two | Solution prepared by mixing 6.5ml Bovine IgG (10mg/ml, pH 7.4) and 13.5ml aqueous DL-Valine (59.2mg/ml, pH 6.2) @ 1.25 ml/min | Propan-2-ol saturated with D,L-valine @ 23.75 ml min⁻¹ | - | 25 ml min⁻¹ |
| Three | Bovine IgG (9.72 mg/ml, pH 7.4) @ 0.42 ml/min | D, L-valine (60 mg/ml in deionized water @ 0.83 ml min⁻¹) | Propan-2-ol saturated with D,L-valine @ 23.75 ml min⁻¹ | 25 ml min⁻¹ |

Table 8 shows the differences in the amount of soluble protein that can be recovered from the formulations following storage as a dry powder at room temperature for 1 day and 6 days. It is clear that the 3 line process provides a significant advantage in terms of retention of protein integrity - there is no detectable protein aggregation following processing and the formulation is more stable to storage at room temperature.

Table 8

| Sample | Theoretical Load (% w/w) | LOADING of soluble protein (%w/w) determined from UV 280 | Solubility (%) |
|---|---|---|---|
| 3 LINE (analysed after 1 day) | 7.5 | 7.7 | 103 |
| 2 LINE (analysed after 1 day) | 7.5 | 6.6 | 88 |
| | | | |
| 3 LINE (analysed after 6 days) | 7.5 | 5.8 | 77 |
| 2 LINE (analysed after 6 days) | 7.5 | 3.7 | 49 |

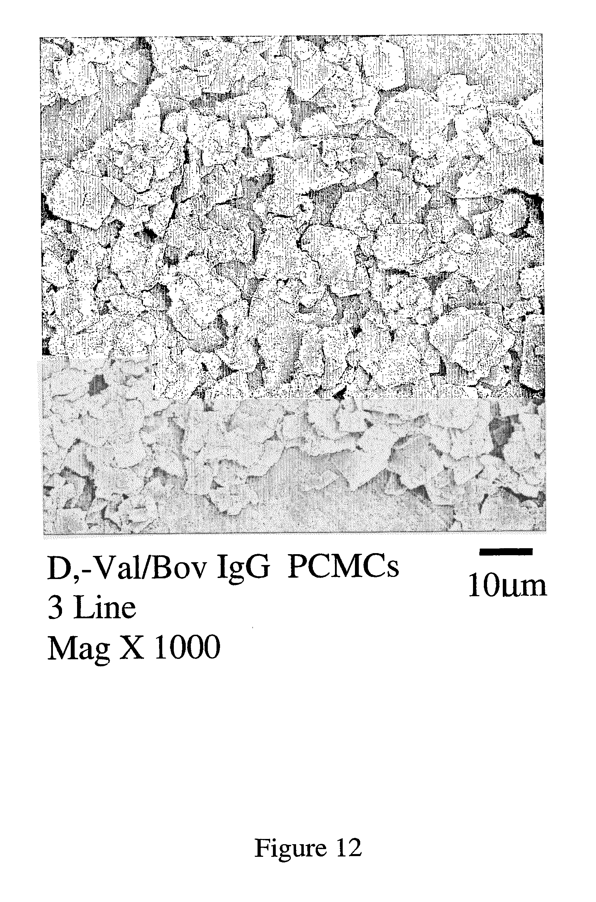

Figure 12 shows an SEM image of the Bovine IgG coated microcrystals prepared by the 3 line system. The observed flakes have a diameter of 5-15 microns. This type of flake has been found previously to be suitable for delivery by dry powder inhalers and to exhibit aerodynamic mass median diameters less than the geometric diameter (composition patent). It is expected that these formulations will therefore be well suited for delivering antibodies and Fc-fusion proteins and Fc-drug congugates to the central airways for systemic delivery via active transport.

Example 5

Trypsin coated glutamine microcrystals were prepared by the 2 line process and 3 line process using the equipment and suppliers described in Example 1 and conditions described in Table x, (Trypsin (Sigma Cat# T1426, Lot 104K7575), L-glutamine (Sigma Cat# G8540, Lot 072K03651)).

Thus, the aqueous solution containing the protein was either pH 6.3 as set by the glutamine in the 2 line system or else pH 9.2 as set by the phosphate buffer in the 3 line system. A pH of 9.2 is close to the pI of trypsin. In the 3 line system the pH of the glutamine solution was again pH 6.3. The crystals produced were collected, rinsed with solvent, dried and assayed for trypsin bioactivity using the hydrolysis assay described in Example 1.

The results are shown in Table 9.

Table 9

| Number of Lines | Pump A Solute & Flow Rate | Pump B Solute & Flow Rate | Pump C Solute & Flow Rate | Total Flow Rate |
|---|---|---|---|---|
| Two | Trypsin (2.35mg/ml) + L-glutamine (13.3 mg/ml) in deionized water @ 1 ml min$^{-1}$ | Propan-2-ol saturated with L-glutamine @ 24 ml min$^{-1}$ | - | 25 ml min$^1$ |
| Three | Trypsin (7.00 mg/ml) in pH 9.2 0.01M potassium phosphate @ 0.335 ml min$^{-1}$ | L-glutamine (20 mg/ml in deionized water @ 0.665 ml min$^{-1}$) | Propan-2-ol saturated with L-glutamine @ 24 ml min$^{-1}$ | 25 ml min$^{-1}$ |

The percentage of retained bioactivity was calculated relative to the maximum expected if all the protein had been immobilized.

As shown in Table 10 the sample prepared by the 3 line system (with the pH of the protein close to the pI of trypsin) lead to a significantly higher retention of bioactive trypsin than in the 2 line system. RP-HPLC confirmed that this difference correlated with a change in the amount of protein bound to the microcrystal. The 3 line system therefore provides a way of obtaining improved efficiency of binding with introduction of lower amounts of buffer into the product.

Table 10

| Number of Lines | Sample reference | Retained bioactivity (%) |
|---|---|---|
| 2 | JV1195/1 | 14.7 |

(continued)

| Number of Lines | Sample reference | Retained bioactivity (%) |
|---|---|---|
| 3 | JV1195/2 | 85.4 |

**Example 6**

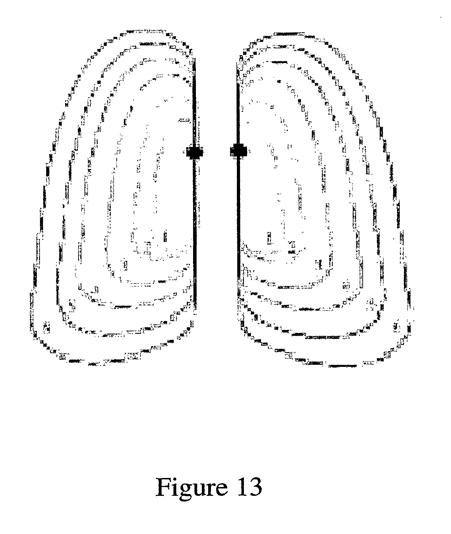

Figure 13 is an image showing different parts of a lung with regions 1 to 6.

Regions 5 and 6 are known as the alveolar space in the lungs. Microcrystals with a particle size of about 1 micron may also be delivered to region 6 via a pulmonary device. Microcrystals with a particle size of about 1 - 5 micron may be delivered to region 5 via a pulmonary device.

Regions 3 and 4 are known as the large ciliated airways space in the lungs. Microcrystals with a particle size of about 5 - 10 microns may be delivered to regions 3 and 4 via a pulmonary device.

Regions 1 and 2 are known as the upper respiratory tract and in most situations it is not suitable to provide microcrystals in this region due to lack of uptake.

By adapting the process of manufacturing the bioactive molecule coated microcrystals, the microcrystals may be adapted so that they preferentially bind in any of regions 1 to 6 or combination thereof as shown in Figure 13.

Parameters that may affect the size of particles include concentration of coprecipitant, pH, solvent, water content, bioactive molecule loading, temperature, additive concentration and mixing efficiency. If sequential addition of the first and second aqueous solutions to the solvent is used the time for the solution to pass from the first mixer to the second mixer may also be varied. In some systems one or more of these values may necessarily be fixed but DoE may be used to carry out an efficient screen of the remaining variable parameters. It can be used to identify those that most affect the size and any interactions between them. This information can then be used to further refine the parameters via response surface modelling to generate a particle size within a target range while at the same time also optimising bioactivity and the efficiency of binding.

**Claims**

1. A continuous method of forming bioactive molecule coated microcrystals comprising the following steps:

   (a) providing a first aqueous solution comprising coprecipitant molecules;
   (b) providing a second aqueous solution comprising bioactive molecules;
   (c) providing a third solution comprising water miscible solvent;
   (d) mixing said first aqueous solution, said second aqueous solution and said third solution simultaneously; wherein a first pump continuously delivers the first aqueous solution comprising coprecipitant molecules, a second pump continuously delivers the second aqueous solution comprising bioactive molecules and a third pump continuously delivers the third solution comprising water miscible solvent, such that coprecipitation of the coprecipitant and the bioactive molecules is initiated leading to formation of said microcrystals; and
   (e) collecting a suspension of microcrystals.

2. A method according to claim 1, wherein the water miscible solvents are short-chain alcohols such as methanol; ethanol; propan-1-ol; propan-2-ol; aldehydes or ketones such as acetone, esters such as ethyl lactate, ethers such as tetrahydrofuran, diols such as 2-methyl-2,4- pentanediol, 1,5-pentane diol, and various size polythene glycol (PEGS) and polyols; or any combination or mixture thereof.

3. The method according to either of claims 1 or 2 wherein the mixing occurs in a static or dynamic mixing device.

4. A method according to any preceding claim, wherein the aqueous solutions are delivered at flow rates between 0.2ml/min and about 1000ml/min.

5. A pharmaceutical formulation comprising particles that contain one or more microcrystals wherein the microcrystals comprise:

   (a) a substantially non-hydroscopic inner crystalline core formed from coprecipitant molecules; and
   (b) an outer coating comprising one or more bioactive molecules;
   wherein the coated microcrystals have been formed in a single continuous process according to Claim 1 by mixing a first aqueous solution comprising coprecipitant molecules, a
second solution comprising bioactive molecules, and a third solution comprising water miscible solvent simultaneously.

6. A pharmaceutical formulation according to claim 5, wherein the outer coating thickness ranges from 0.01 to 1000 microns, 1 to 100 microns, 5 to 50 microns or 10 to 20 microns.

7. A pharmaceutical formulation according any of claims 5 or 6, wherein the particles have a maximum cross-sectional dimension of less than $80\mu m$, less than $50\mu m$ across or less than $20\mu m$.

8. A pharmaceutical formulation according to any of claim 5 to 7, wherein the molecules making up the crystalline core have a molecular weight less than 2kDa.

9. A pharmaceutical formulation according to any of claims 5 to 8, wherein the molecules which make up the crystalline core are selected from any of: amino acids, zwitterions, peptides, sugars, buffer components, water soluble drugs, organic and inorganic salts, compounds that form strongly hydrogen bonded lattices or derivatives or any combination thereof.

10. A pharmaceutical formulation according to any of claims 5 to 9, comprising drugs such as: anti-inflammatories; anti-cancer; anti-psychotic; anti-bacterial; anti-fungal; natural or unnatural peptides; proteins such as insulin, $\alpha$1-antitrypsin, $\alpha$-chymotrypsin, albumin; interferons; antibodies; fusion proteins such as Fc-fusion proteins; protein-drug conjugates, nucleic acids such as fragments of genes, DNA, RNA or RNAi from natural sources or synthetic oligonucleotides and anti-sense nucleotides; sugars such as any mono-, di- or polysaccharides; plasmids; and protein/peptide-drug conjugates.

11. A pharmaceutical formulation according to any of claims 5 to 10, wherein the particles include those with a crystalline core of D,L-valine and a coating of insulin; a crystalline core of L-glycine and a coating of antitrypsin, a crystalline core of Na glutamate and a coating of insulin; a crystalline core of L-methionine and a coating of insulin; a crystalline core of L-alanine and a coating of insulin; a crystalline core of L-valine and a coating of insulin; a crystalline core of L-histidine and a coating of insulin; a crystalline core of L-glycine and a coating of $\alpha$ - antitrypsin; a crystalline core of L-glutamine and a coating of albumin: a crystalline core of D, L-valine and a coating of oligonucleotides DQA-HEX; a crystalline core of D,L-valine and a coating of $\alpha$1-antitrypsin with a further anti-oxidant outer coating of N-acetyl cystein; a crystalline core of D,L-valine and a coating of ovalbumin; a crystalline core of L-glutamine and a coating of ovalbumin, a crystalline core D,L-valine and a coating of diphtheria taxoid; a crystalline core of L-glutamine and a coating of diphtheria taxoid; a crystalline core of D,L-valine and a coating of diphtheria taxoid; a crystalline core of L-glutamine and a coating of tetanus taxoid; a crystalline core of D,L-valine and a coating of a mixture of diphtheria taxoid and tetanus taxoid; a crystalline core of L-glutamine and a coating of a mixture of diphtheria taxoind and tetanus taxoid.

12. A pharmaceutical formulation for pulmonary delivery comprising microcrystals formed according to any of claims 1 to 11.

13. A pharmaceutical formulation according to claim 12, wherein bioactive molecules for the formation of pulmonary pharmaceutical formulations are selected from any of the following: therapeutic proteins such as insulin, $\alpha$1-antitrypsin, interferons; antibodies and antibody fragments and derivatives; therapeutic peptides and hormones; synthetic and natural DNA including DNA based medicines; enzymes; vaccine components; antibiotics; pain-killers; water-soluble drugs; water-sensitve drugs; lipids and surfactants; polysaccharides; or any combination of derivatives thereof.

14. A pharmaceutical formulation according to any of claims 12 or 13, wherein the pulmonary formulations comprise particles with a mass median aerodynamic diameter less than 10 microns or less than 5 microns.

15. A pharmaceutical formulation according to any of claims 12 to 14, wherein the pulmonary formulations have crystalline cores comprised of amino-acids such as valine, histidine, isoleucine, glycine or glutamine and which, for example, include: a crystalline core of valine and a coating of a therapeutic protein such as insulin; a crystalline core of histidine and a coating of an enzyme; a crystalline core of valine and a coating of an enzyme inhibitor such as $\alpha$-antitrypsin; a crystalline core of valine and a coating of DNA; a crystalline core of valine and a vaccine coating; a crystalline core of glutamine and a vaccine coating; a crystalline core of glutamine and a coating of albumin.

16. A parenteral formulation comprising microcrystals or suspension of microcrystals formed according to any claims 1 to 4.

17. A sustained or controlled release pharmaceutical formulation, or a depots, comprising microcrystals or suspensions of microcrystals formed according to any of claims 1 to 4.

18. A drug delivery device for inhalation comprising microcrystals formed according to any of claims 1 to 4.

**Patentansprüche**

1. Kontinuierliches Verfahren zur Bildung von mit bioaktiven Molekülen beschichteten Mikrokristallen, das folgende Schritte umfasst:

   (a) das Bereitstellen einer ersten wässrigen Lösung, die Moleküle eines Co-Fällungsmittels umfasst;
   (b) das Bereitstellen einer zweiten wässrigen Lösung, die bioaktive Moleküle umfasst;
   (c) das Bereitstellen einer dritten Lösung, die ein wassermischbares Lösungsmittel umfasst;
   (d) das gleichzeitige Vermischen der ersten wässrigen Lösung, der zweiten wässrigen Lösungen und der dritten Lösung; wobei eine erste Pumpe kontinuierlich die erste wässrige Lösung liefert, die Moleküle eines Co-Fällungsmittels umfasst, eine zweite Pumpe kontinuierlich die zweite wässrige Lösung liefert, die bioaktive Moleküle umfasst und eine dritte Pumpe kontinuierlich die dritte Lösung liefert, die ein wassermischbares Lösungsmittel umfasst, sodass die Co-Fällung des Co-Fällungsmittels und der bioaktiven Moleküle eingeleitet wird, was zur Bildung der Mikrokristalle führt; und
   (e) das Sammeln einer Suspension von Mikrokristallen.

2. Verfahren nach Anspruch 1, wobei die wassermischbaren Lösungsmittel kurzkettige Alkohole wie Methanol; Ethanol; 1-Propanol; 2-Propanol; Aldehyde oder Ketone wie Aceton, Ester wie Ethyllactat, Ether wie Tetrahydrofuran, Diole wie 2-Methyl-2,4-Pentandiol, 1,5-Pentandiol sowie Polyethylenglykol (PEGS) und Polyole mit verschiedenen Größen sind; oder eine beliebige Kombination bzw. ein beliebiges Gemisch davon.

3. Verfahren nach Anspruch 1 oder 2, wobei das Vermischen in einer statischen oder dynamischen Mischvorrichtung stattfindet.

4. Verfahren nach einem beliebigen der vorhergehenden Ansprüche, wobei die wässrigen Lösungen bei Durchflussgeschwindigkeiten zwischen 0,2 ml/min und ungefähr 1000 ml/min geliefert werden.

5. Pharmazeutische Formulierung, die Partikel umfasst, die einen oder mehrere Mikrokristalle beinhalten, wobei die Mikrokristalle Folgendes umfassen:

   (a) einen im Wesentlichen nicht hygroskopischen inneren kristallinen Kern, der aus Molekülen eines Co-Fällungsmittels gebildet wird; und
   (b) eine äußere Beschichtung, die ein oder mehrere bioaktive Moleküle umfasst;
   wobei die beschichteten Mikrokristalle in einem einzigen kontinuierlichen Verfahren nach Anspruch 1, durch gleichzeitiges Vermischen einer ersten wässrigen Lösung, die Moleküle eines Co-Fällungsmittels umfasst, eine zweiten Lösung, die bioaktive Moleküle umfasst und einer dritten Lösung, die ein wassermischbares Lösungsmittel umfasst, gebildet werden.

6. Pharmazeutische Formulierung nach Anspruch 5, wobei die Dicke der äußeren Beschichtung von 0,01 bis 1000 Mikrometer, von 1 bis 100 Mikrometer, von 5 bis 50 Mikrometer oder von 10 bis 20 Mikrometer reicht.

7. Pharmazeutische Formulierung nach einem beliebigen der Ansprüche 5 oder 6, wobei die Partikel eine maximale Querschnittsabmessung von weniger als 80 $\mu$m, weniger als 50 $\mu$m quer oder weniger als 20 $\mu$m aufweisen.

8. Pharmazeutische Formulierung nach einem beliebigen der Ansprüche 5 bis 7, wobei die Moleküle, aus denen der kristalline Kern besteht, eine molekulare Masse von weniger als 2 kDa aufweisen.

9. Pharmazeutische Formulierung nach einem beliebigen der Ansprüche 5 bis 8, wobei die Moleküle, aus denen der kristalline Kern besteht, aus einem der Folgenden ausgewählt werden: Aminosäuren; Zwitterionen; Peptide; Zucker;

Pufferkomponenten; wasserlösliche Arzneimittel; organische und anorganische Salze; Verbindungen, die fest über Wasserstoffbrückenbindungen verbundene Gitter formen oder Derivaten oder einer beliebigen Kombination davon.

10. Pharmazeutische Formulierung nach einem beliebigen der Ansprüche 5 bis 9, welche Arzneimittel umfasst wie etwa: Entzündungshemmer; Krebsmedikamente; Antipsychotika; antibakterielle Medikamente; Antimykotika; natürliche oder künstliche Peptide; Proteine wie Insulin, $\alpha$1-Antitrypsin, $\alpha$-Chymotrypsin, Albumin; Interferone; Antikörper; Fusionsproteine wie Fc-Fusionsproteine; Konjugate von Proteintherapeutika, Nukleinsäuren wie Fragmente von Genen, DNA, RNA oder RNAi aus natürlichen Quellen oder synthetische Oligonukleotide und Antisense-Nukleotide; Zucker wie beliebige Mono-, Di-, oder Polysaccharide; Plasmide; und Konjugate von Protein-/Peptidtherapeutika.

11. Pharmazeutische Formulierung nach einem beliebigen der Ansprüche 5 bis 10, wobei die Partikel diejenigen umfassen mit einem kristallinen Kern aus D,L-Valin und einer Beschichtung aus Insulin; einem kristallinen Kern aus L-Glycin und einer Beschichtung aus Antitrypsin; einem kristallinen Kern aus Na-Glutamat und einer Beschichtung aus Insulin; einem kristallinen Kern aus L-Methionin und einer Beschichtung aus Insulin; einem kristallinen Kern aus L-Alanin und einer Beschichtung aus Insulin; einem kristallinen Kern aus L-Valin und einer Beschichtung aus Insulin; einem kristallinen Kern aus L-Histidin und einer Beschichtung aus Insulin; einem kristallinen Kern aus L-Glycin und einer Beschichtung aus $\alpha$-Antitrypsin; einem kristallinen Kern aus L-Glutamin und einer Beschichtung aus Albumin; einem kristallinen Kern aus D,L-Valin und einer Beschichtung aus Oligonukleotiden DQA-HEX; einem kristallinen Kern aus D,L-Valin und einer Beschichtung aus $\alpha$1-Antitrypsin mit einer weiteren antioxidativen Beschichtung aus N-Acetylcystein; einem kristallinen Kern aus D,L-Valin und einer Beschichtung aus Ovalbumin; einem kristallinen Kern aus L-Glutamin und einer Beschichtung aus Ovalbumin; einem kristallinen Kern aus D,L-Valin und einer Beschichtung aus Diphterie-Toxoid; einem kristallinen Kern aus L-Glutamin und einer Beschichtung aus Diphterie-Toxoid; einem kristallinen Kern aus L-Glutamin und einer Beschichtung aus Tetanustoxoid; einem kristallinen Kern aus D,L-Valin und einer Beschichtung aus einem Gemisch aus Diphterie-Toxoid und Tetanustoxoid; einem kristallinen Kern aus L-Glutamin und einer Beschichtung aus einem Gemisch aus Diphterie-Toxoid und Tetanustoxoid.

12. Pharmazeutische Formulierung für die pulmonale Zuführung, die Mikrokristalle umfasst, welche nach einem beliebigen der Ansprüche 1 bis 11 gebildet werden.

13. Pharmazeutische Formulierung nach Anspruch 12, wobei bioaktive Moleküle für die Bildung pulmonaler pharmazeutischer Formulierungen aus einem der Folgenden ausgewählt werden: therapeutische Proteine wie Insulin, $\alpha$1-Antitrypsin, Interferone; Antikörper und Antikörperfragmente und -derivate; therapeutische Peptide und Hormone; synthetische und natürliche DNA einschließlich DNA-basierter Arzneimittel; Enzyme; Impfstoffkomponenten; Antibiotika; Schmerzmittel; wasserlösliche Arzneimittel; wasserempfindliche Arzneimittel; Lipide und Tenside; Polysaccharide; oder eine beliebige Kombination bzw. Derivate davon.

14. Pharmazeutische Formulierung nach einem beliebigen der Ansprüche 12 oder 13, wobei die pulmonalen Formulierungen Partikel mit einem massenbezogenen medianen Durchmesser von weniger als 10 Mikrometer oder weniger als 5 Mikrometer umfassen.

15. Pharmazeutische Formulierung nach einem beliebigen der Ansprüche 12 bis 14, wobei die pulmonalen Formulierungen kristalline Kerne aufweisen, die aus Aminosäuren wie Valin, Histidin, Isoleucin, Glycin oder Glutamin bestehen und welche zum Beispiel Folgendes umfassen: einen kristallinen Kern aus Valin und eine Beschichtung aus einem Proteintherapeutikum wie Insulin; einen kristallinen Kern aus Histidin und eine Beschichtung aus einem Enzym; einen kristallinen Kern aus Valin und eine Beschichtung aus einem Enzyminhibitor wie $\alpha$-Antitrypsin; einen kristallinen Kern aus Valin und eine Beschichtung aus DNA; einen kristallinen Kern aus Valin und eine Beschichtung aus einem Impfstoff; einen kristallinen Kern aus Glutamin und eine Beschichtung aus einem Impfstoff; einen kristallinen Kern aus Glutamin und eine Beschichtung aus Albumin.

16. Parenterale Formulierung, die Mikrokristalle oder eine Suspension von Mikrokristallen umfasst, die nach einem beliebigen der Ansprüche 1 bis 4 gebildet werden.

17. Pharmazeutische Formulierung oder Depot mit retardierter oder kontrollierter Wirkstofffreigabe, welche(s) Mikrokristalle oder Suspensionen von Mikrokristallen umfasst, die nach einem beliebigen der Ansprüche 1 bis 4 gebildet werden.

**18.** Vorrichtung zur Arzneimittelabgabe für die Inhalation, welche Mikrokristalle umfasst, die nach einem beliebigen der Ansprüche 1 bis 4 gebildet werden.

**Revendications**

**1.** Procédé continu de formation de microcristaux revêtus de molécules bioactives comprenant les phases suivantes:

(a) fourniture d'une première solution aqueuse des molécules de co-précipitation;
(b) fourniture d'une deuxième solution aqueuse des molécules bioactives;
(c) fourniture d'une troisième solution comprenant un solvant miscible dans l'eau ;
(d) mélange de ladite première solution aqueuse, de ladite deuxième solution aqueuse et de ladite troisième solution simultanément ; où une première pompe délivre en continu la première solution aqueuse comprenant des molécules de co-précipitation, une deuxième pompe délivre en continu la deuxième en solution comprenant des molécules bioactives et une troisième pompe délivre en continu la troisième solution comprenant un solvant miscible dans l'eau, de sorte que la co-précipitation du co-précipitant et des molécules bioactives est initiée, conduisant à la formation desdits microcristaux ; et
(e) recueil d'une suspension de microcristaux.

**2.** Procédé selon la revendication 1, dans lequel les solvants miscibles dans l'eau sont des alcools à courte chaîne comme le méthanol; l'éthanol; le propan-1-ol; le propan-2-ol; les aldéhydes ou les cétones comme l'acétone, les esters comme le lactate éthylique, les éthers comme le tétrahydrofurane, les diols comme le 2-méthyle-2, le 4-pentanediol, le 1,5-pentane diol et les polyéthylènes glycols (PEGS) et les polyols de diverse taille; ou toute combinaison ou leurs mélanges.

**3.** Le procédé selon l'une quelconque des revendications 1 ou 2, où le mélange se produit dans un dispositif de gâchage statique ou dynamique.

**4.** Procédé selon l'une quelconque des revendications précédentes, où les solutions aqueuses sont délivrées à des débits compris entre 0,2 ml/min et environ 1000 ml/min.

**5.** Formulation pharmaceutique comprenant des particules contenant un ou plusieurs microcristaux où les microcristaux comprennent :

(a) un noyau cristallin interne sensiblement non hydroscopique formé à partir de molécules de co-précipitation; et
(b) un revêtement externe comprenant une ou plusieurs molécules bioactives;
où les microcristaux revêtus ont été formés lors d'un simple procédé continu selon la revendication 1 en mélangeant une première solution aqueuse comprenant des molécules de co-précipitation,
une deuxième solution comprenant des molécules bioactives et une troisième solution comprenant un solvant miscible dans l'eau simultanément.

**6.** Formulation pharmaceutique selon la revendication 5, où l'épaisseur du revêtement externe est comprise entre 0,01 et 1000 microns, 1 et 100 microns, 5 et 50 microns ou bien 10 et 20 microns.

**7.** Formulation pharmaceutique selon l'une quelconque des revendications 5 ou 6, où les particules ont une section maximale inférieure à 80 $\mu$m, inférieure à 50 $\mu$m ou inférieure à 20 $\mu$m.

**8.** Formulation pharmaceutique selon l'une quelconque des revendications 5 à 7, où les molécules composant le noyau cristallin ont un poids moléculaire inférieur à 2 kDa.

**9.** Formulation pharmaceutique selon l'une quelconque des revendications 5 à 8, où les molécules composant le noyau cristallin sont sélectionnés parmi l'un des éléments suivants : les acides aminés, les zwittérions, les peptides, les sucres, les composants tampons, les médicaments solubles dans l'eau et les sels inorganiques, les composés constituant des treillis à forte liaison d'hydrogène ou bien les dérivés ou les combinaisons de ceux-ci.

**10.** Formulation pharmaceutique selon l'une quelconque des revendications 5 à 9, comprenant des médicaments comme les suivants :

les anti-inflammatoires ; les anticancéreux ; les antipsychotiques ; les antibactériens ; les antifongiques ; les peptides naturels ou non naturels ; les protéines comme l'insuline, l'a1-antitrypsine, l'a-chymotrypsine, l'albumine ; les interférons; les anticorps; les protéines de fusion comme les protéines de fusion Fc ; les conjugués de médicament et protéine, les acides nucléique comme les fragments de gènes, l'ADN, l'ARN ou l'iARN à partir d'oligonucléotides synthétiques ou de source naturelle et de nucléotides antisens; les sucres comme les mono-, di- or polysaccharides ; les plasmides; et les conjugués de protéine/de médicament et peptide.

11. Formulation pharmaceutique selon l'une quelconque des revendications 5 à 10, où les particules englobent les particules avec un noyau cristallin de D,L-valine et un revêtement d'insuline ; un noyau cristallin de L-glycine et un revêtement d'antitrypsine, un noyau cristallin et Na-glutamate et un revêtement d'insuline ; un noyau cristallin de L-méthionine et un revêtement d'insuline ; un noyau cristallin de L-alanine et un revêtement d'insuline ; un noyau cristallin de L-valine et un revêtement d'insuline ; un noyau cristallin de L-histidine et un revêtement d'insuline ; un noyau cristallin de L-glycine et un revêtement d'a-antitrypsine; un noyau cristallin de L-glutamine et un revêtement d'albumine; un noyau cristallin de D, L-valine et un revêtement d'oligonucléotides DQA-HEX ; un noyau cristallin de D, L-valine et un revêtement d'a1-antitrypsine avec un revêtement externe antioxydant supplémentaire de cystéine N-acétylique ; un noyau cristallin de D,L-valine et un revêtement d'ovalbumine; un noyau cristallin de L-glutamine et un revêtement d'ovalbumine; un noyau cristallin de D,L-valine et un revêtement d'anatoxine diphtérique ; un noyau cristallin de L-glutamine et un revêtement d'anatoxine diphtérique ; un noyau cristallin de D,L-valine et un revêtement d'anatoxine diphtérique ; un noyau cristallin de L-glutamine et un revêtement d'anatoxine tétanique; un noyau cristallin de D,L-valine et un revêtement composé d'un mélange d'anatoxine diphtérique et d'anatoxine tétanique; un noyau cristallin de la L-glutamine et un revêtement composé d'un mélange d'anatoxine diphtérique et d'anatoxine tétanique

12. Formulation pharmaceutique pour administration pulmonaire comprenant des microcristaux selon l'une quelconque des revendications 1 à 11.

13. Formulation pharmaceutique selon la revendication 12, où les molécules bioactives pour la formation de formulation pharmaceutiques pulmonaires sont sélectionnées parmi les molécules suivantes : les protéines thérapeutiques comme l'insuline, l'a1-antitrypsine, les interférons; les anticorps et les fragments d'anticorps et les dérivés; les peptides et hormones thérapeutiques ; l'ADN synthétique et naturel y compris les médicaments à base d'ADN ; les enzymes, les composants de vaccin ; les antibiotiques ; les médicaments antidouleur ; les médicaments solubles dans l'eau ; les médicaments sensibles à l'eau ; les lipides et les tensioactifs; les polysaccharides ; ou toute combinaison de leurs dérivés.

14. Formulation pharmaceutique selon l'une quelconque des revendications 12 ou 13, où les formulations pulmonaires comprennent des particules d'un diamètre aérodynamique moyen en masse inférieur à 10 microns ou inférieur à 5 microns.

15. Formulation pharmaceutique selon l'une quelconque des revendications 12 à 14, où les formulations pulmonaires ont des noyaux cristallins composés d'acides aminés comme la valine, l'histidine, l'isoleucine, la glycine ou la glutamine et qui, par exemple, englobent : un noyau cristallin de L-valine et un revêtement de protéine thérapeutique comme l'insuline; un noyau cristallin d'histidine et un revêtement composé d'un enzyme ; un noyau cristallin de valine et un revêtement composé d'un inhibiteur d'enzyme comme l'antitrypsine; un noyau cristallin de L-valine et un revêtement d'ADN ; un noyau cristallin de L-valine et un revêtement de vaccin; un noyau cristallin de glutamine et un revêtement de vaccin; un noyau cristallin de glutamine et un revêtement d'albumine.

16. Formulation parentérale comprenant des microcristaux ou une suspension de microcristaux selon l'une quelconque des revendications 1 à 4.

17. Formulation pharmaceutique à libération contrôlée ou continue, ou dépôt, comprenant des microcristaux ou des suspensions de microcristaux selon l'une quelconque des revendications 1 à 4.

18. Dispositif d'administration de médicament pour inhalation comprenant des microcristaux formés selon l'une quelconque des revendications 1 à 4.

## BCA Protein
## Loading (%w/w)

**Measured
Protein
Loading
(%w/w)**

Figure 1

**TAME % Retained Activity**

Figure 2

**BCA Corrected TAME**
**% Retained Activity**

Figure 3

**Size & Span Measurements of
Trypsin-DL-valine PCMCs**

Figure 4

Figure 5

Figure 6

Figure 7

Figure 8

Figure 9

Figure 10

Figure 11

D,-Val/Bov IgG  PCMCs
3 Line
Mag X 1000

10um

Figure 12

Figure 13

**EP 1 778 194 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0069887 A
- US 5662883 A
- WO 0141811 A
- US 5807746 A
- WO 9905302 A
- GB 2004000044 W